# EUROPEAN PATENT APPLICATION

(11) **EP 1 741 457 A1**
(43) Date of publication of application: **10.01.2007**
(21) Application number: 06016006.6
(22) Date of filing: 11.08.2003
(51) Int. Cl.: A61L 27/26, A61L 27/34, A61L 27/38, A61F 2/14, C08F 220/54, C08G 81/00

(54) **Ocular implant**

(30) Priority: 09.08.2002 CA 2397379
(62) Divisional of application: 03783866.1
(71) Applicant: Ottawa Health Research Institute, Ottawa, Ontario K1Y 4E9 (CA); NATIONAL RESEARCH COUNCIL OF CANADA, Ottawa, Ontario K1A 0R6 (CA)
(72) Inventor: Carlsson, David J., Ottawa Ontario K1J 7E6 (CA); Griffith, May, Carp Ontario K0A 1L0 (CA); Li, Fengfu, Ottawa Ontario K1J 1J1 (CA)
(74) Representative: Bentham, Andrew

(57) **Abstract**

A bio-synthetic matrix comprising a hydrogel which is formed by cross-linking a synthetic polymer and a bio-polymer is provided. The matrix is robust, biocompatible and non-cytotoxic and is capable of supporting cell in growth *in vivo.* The matrix can be tailored to further comprise one or more bioactive agents. The matrix may also comprise cells encapsulated and dispersed therein, which are capable of proliferating upon deposition of the matrix *in vivo.* Methods of preparing the bio-synthetic matrix and the use of the matrix *in vivo* for tissue engineering or drug delivery applications are also provided.

## Description

### FIELD OF THE INVENTION

The present invention pertains to the field of tissue engineering and in particular to a bio-synthetic matrix comprising a hydrogel suitable for *in vivo* use.

### BACKGROUND

Tissue engineering is a rapidly growing field encompassing a number of technologies aimed at replacing or restoring tissue and organ function. The key objective in tissue engineering is the regeneration of a defective tissue through the use of materials that can integrate into the existing tissue so as to restore normal tissue function. Tissue engineering, therefore, demands materials that can support cell over-growth, in-growth or encapsulation and, in many cases, nerve regeneration.

Polymer compositions are finding widespread application in tissue engineering. Natural bio-polymers such as collagens, fibrin, alginates and agarose are known to be non-cytotoxic and to support over-growth, in-growth and encapsulation of living cells. Matrices derived from natural polymers, however, are generally insufficiently robust for transplantation. In contrast, matrices prepared from synthetic polymers can be formulated to exhibit predetermined physical characteristics such as gel strength, as well as biological characteristics such as degradability. Reports that synthetic analogues of natural polymers, such as polylysine, poly(ethylene imine), and the like, can exhibit cytotoxic effects [Lynn & Langer, J. Amer. Chem. Soc., 122:10761-10768 (2000)] have lead to the development of alternative synthetic polymers for tissue engineering applications.

Hydrogels are crosslinked, water-insoluble, water-containing polymers which offer good biocompatibility and have a decreased tendency to induce thrombosis, encrustation, and inflammation and as such are ideal candidates for tissue engineering purposes. The use of hydrogels in cell biology is well known [see, for example, A. Atala and R.P. Lanza, eds., "Methods in Tissue Engineering" Academic Press, San Diego, 2002]. A wide variety of hydrogels for *in vivo* applications have been described [see, for example, the review by Jeong, et al., Adv. Drug Deliv. Rev., 54:37-51 (2002)]. Hydrogels based on N-isopropylacrylamide (NiPAAm) and certain co-polymers thereof, for example, are non-toxic and capable of supporting growth of encapsulated cells *in vitro* [Vernon, et al., Macromol. Symp., 109:155-167 (1996); Stile, et al., Macromolecules, 32:7370-9 (1999); Stile, et al.,Biomacromolecules 3: 591- 600. (2002*);* Stile, et al., Biomacromolecules 2: 185-194.(2001*);* Webb, et al., MUSC Orthopaedic J., 3:18-21 (2000); An et al., U.S. Patent No. 6,103,528]. Temperature-sensitive NiPAAm polymers have also been described for use in immunoassays [U.S. Patent No. 4,780,409]. However, despite manipulations of synthesis conditions and improvements to enhance biocompatibility, it is still difficult to obtain a seamless host-implant interface and complete integration of the hydrogel implant into the host [Hicks, et al. Surv. Ophthalmol. 42: 175-189 (1997); Trinkaus-Randall and Nugent, J. Controlled Release 53:205-214 (1998)].

Modifications of synthetic polymer gels with a second naturally derived polymer to generate an interpenetrating polymer network ("IPN") structure have been reported [For example, see Gutowska et al., Macromolecules, 27:4167 (1994); Yoshida et al., Nature, 374:240 (1995); Wu & Jiang, U.S. Patent No. 6,030,634; Park et al., U.S. Patent No. 6,271,278]. However, these structures are frequently destabilised by extraction of the naturally derived component by culture media and by physiological fluids. Naturally derived polymers also tend to biodegrade rapidly within the body resulting in destabilisation of *in vivo* implants.

More robust hydrogels comprising cross-linked polymer compositions have also been described. For example, U.S. Patent No. 6,388,047 describes a composition consisting of a hydrophobic macromer and a hydrophilic polymer that are cross-linked to form a hydrogel by free-radical polymerisation. U.S. Patent No. 6,323,278 describes a cross-linked polymer composition which can form *in situ* and which comprises two synthetic polymers, containing multiple electrophilic groups and the other containing multiple nucleophilic groups. Both U.S. Patent No. 6,388,047 and 6,384,105 describe systems that must be cross-linked by free radical chemistry, which requires the use of initiators that are well known to be cytotoxic (azo compounds, persulfates), thus leading to possible side effects if the hydrogel was to be used in the tissue or with encapsulated cells.

U.S. Patent No. 6,384,105 describes injectable, biodegradable polymer composites comprising poly(propylene fumarate) and poly(ethylene glycol)-dimethacrylate which can be cross-linked *in situ.* The hydrogels described in this patent are largely based on polymers with a polyethylene oxide backbone polymers. Although these polymers are known to be biocompatible, their ability to support cell growth is uncertain.

U.S. Patent No. 6,566,406 describes biocompatible cross-linked hydrogels that are formed from water soluble precursors having electrophilic and nucleophilic groups capable of reacting and cross-linking *in situ.* The precursors are described as being a polyalkylene oxide polymer and a cross-linker. As indicated above, the ability of polyalkylene oxide backbone polymers to support cell growth is uncertain.

There remains a need therefore, for an improved matrix that is biocompatible, sufficiently robust to function as an implant and that can support cell growth *in vivo.*

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

An object of the present invention is to provide a bio-synthetic matrix and uses thereof. In accordance with an aspect of the present invention, there is provided a synthetic co-polymer suitable for the preparation of a bio-synthetic matrix, comprising one or more N-alkyl or N,N-dialkyl substituted acrylamide co-monomer, one or more hydrophilic co-monomer and one or more acryl- or methacryl- carboxylic acid co-monomer derivatised to contain a pendant reactive moiety capable of cross-linking bioactive molecules, said synthetic polymer having a number average molecular mass between about 2,000 and about 1,000,000.

In accordance with another aspect of the invention, there is provided a bio-synthetic matrix comprising the synthetic co-polymer, a bio-polymer and an aqueous solvent, wherein the synthetic co-polymer and bio-polymer are cross-linked to form a hydrogel.

In accordance with another aspect of the invention, there are provided uses of the bio-synthetic matrix as a scaffold for tissue regeneration, for replacement of damaged or removed tissue in an animal, or for coating surgical implants.

In accordance with another aspect of the invention, there are provided compositions comprising: one or more bioactive agent or a plurality of cells; a synthetic co-polymer of the invention; a bio-polymer; and an aqueous solvent.

In accordance with another aspect of the invention, there is provided an implant for use in tissue engineering comprising a pre-formed bio-synthetic matrix, said matrix comprising an aqueous solvent and a bio-polymer cross-linked with a synthetic co-polymer of the invention.

In accordance with another aspect of the invention, there is provided a use of the implant as an artificial cornea.

In accordance with another aspect of the invention, there is provided a process for preparing a synthetic co-polymer comprising: (a) dispersing one or more N-alkyl or N,N-dialkyl substituted acrylamide co-monomer, one or more hydrophilic co-monomer and one or more acryl- or methacryl- carboxylic acid co-monomer derivatised to contain a pendant cross-linkable moiety in a solvent in the presence of an initiator; (b) allowing the N-alkyl or N,N-dialkyl substituted acrylamide co-monomer, hydrophilic co-monomer and acryl- or methacryl- carboxylic acid co-monomer to polymerise to form a synthetic co-polymer, and (c) optionally purifying the synthetic co-polymer; and a process for preparing a bio-synthetic matrix comprising preparing a synthetic co-polymer, dispersing the synthetic co-polymer and a bio-polymer in an aqueous medium and allowing the synthetic co-polymer and the bio-polymer to cross-link to provide the bio-synthetic matrix.

### BRIEF DESCRIPTION OF THE FIGURES

***Figure 1*** depicts the general structure of a terpolymer according to one embodiment of the invention comprising N-isopropylacrylamide, (NiPAAm), acrylic acid (AAc) and N-acryloxysuccinimide (ASI).
***Figure 2*** presents the clinical results from the transplantation into pigs of artificial corneas prepared from a bio-synthetic matrix according to one embodiment of the invention.
***Figure 3*** presents the results of *in vivo* confocal microscopy at 6 weeks post-operative of artificial corneas prepared from a bio-synthetic matrix according to one embodiment of the invention and transplanted into pigs.
***Figure 4*** depicts *in vivo* testing for corneal sensitivity of artificial corneas prepared from a bio-synthetic matrix according to one embodiment of the invention and transplanted into pigs.
***Figures 5, 6 and* 7** present the results of morphological and biochemical assessment of artificial corneas prepared from a bio-synthetic matrix according to one embodiment of the invention and transplanted into pigs.
***Figure 8*** shows (A) the structure of a terpolymer containing a cross-linked bioactive according to one embodiment of the invention, (B) a corneal scaffold composed of cross-linked collagen and the terpolymer shown in (A) and (C) shows a corneal scaffold composed of thermogelled collagen only.
***Figures 9 and 10*** depict the results of delivery of a hydrogel containing collagen and a terpolymer-bioactive agent according to one embodiment of the invention into mouse and rat brains.
***Figure 11*** shows modulus (A) and stress at failure (B) from suture pull out measurements as a function of the concentration ratios of N-acryloxysuccinimde to collagen amine groups for hydrogel matrices according to one embodiment of the invention.
***Figure 12*** depicts transmission (A) and back scattering (B) of light across the visible region as a function of the concentration ratios of N-acryloxysuccinimde to collagen amine groups for hydrogel matrices according to one embodiment of the invention.
***Figure 13*** depicts transmission (A) and back scattering (B) of light across the visible region as a function of the concentration ratios of N-acryloxysuccinimde to collagen amine groups for a hydrogel matrix according to another embodiment of the invention.
***Figure 14*** depicts restoration of touch sensitivity for a pig corneal implant comprising a hydrogel according to one embodiment of the invention.
***Figure 15*** depicts corneal implantation procedure by lamellar keratoplasty in pigs and clinical *in vivo* confocal microscopic images of 6-week implants comprising a hydrogel according to one embodiment of the invention. Bar = 25 µm for D-F, 15 µm for G-O.
***Figure 16*** depicts post-surgical corneal regeneration in pigs receiving corneal implants comprising a hydrogel according to one embodiment of the invention. Bar = 100 µm for A-F, 40 µm for G-I, 200 nm for J-L, 20 µm for M-O, 30 µm for P-R
***Figure 17*** depicts implant-host integration post-surgery at 6 weeks post surgery in pigs receiving corneal implants comprising a hydrogel according to one embodiment of the invention. Bar =100 µm in all cases.
***Figure 18*** depicts corneal touch sensitivity in implants in pigs receiving corneal implants comprising a hydrogel according to one embodiment of the invention.
***Figure 19*** depicts the results of innervation compatibility tests on various hydrogel matrices.
***Figure 20*** depicts epithelial cell growth and stratification on various hydrogels. (A) low magnification views of epithelial growth on the hydrogels (inset is higher magnification) and (B) counts of the cell thickness of the epithelium grown over the hydrogels.

### DETAILED DESCRIPTION OF THE INVENTION

It should be understood that this invention is not limited to the particular process steps and materials disclosed herein, but is extended to equivalents thereof as would be recognised by those ordinarily skilled in the relevant arts. It should also be understood that terminology employed herein is for the purpose of describing particular embodiments only and is not intended to be limiting.

### DEFINITIONS

Unless defined otherwise, all technical and scientific terms used herein have the same meaning as commonly understood by one of ordinary skill in the art to which this invention pertains.

The term "hydrogel," as used herein, refers to a cross-linked polymeric material which exhibits the ability to swell in water or aqueous solution without dissolution and to retain a significant portion of water or aqueous solution within its structure.

The term "polymer," as used herein, refers to a molecule consisting of individual monomers joined together. In the context of the present invention, a polymer may comprise monomers that are joined "end-to-end" to form a linear molecule, or may comprise monomers that are joined together to form a branched structure.

The term "monomer," as used herein, refers to a simple organic molecule which is capable of forming a long chain either alone or in combination with other similar organic molecules to yield a polymer.

The term "co-polymer," as used herein, refers to a polymer that comprises two or more different monomers. Co-polymers can be regular, random, block or grafted. A regular co-polymer refers to a co-polymer in which the monomers repeat in a regular pattern (*e.g.* for monomers A and B, a regular co-polymer may have a sequence: ABABABAB). A random co-polymer is a co-polymer in which the different monomers are arranged randomly or statistically in each individual polymer molecule (e.g. for monomers A and B, a random co-polymer may have a sequence: AABABBABBBAAB). In contrast, a block co-polymer is a co-polymer in which the different monomers are separated into discrete regions within each individual polymer molecule (e.g. for monomers A and B, a block co-polymer may have a sequence: AAABBBAAABBB). A grafted co-polymer refers to a co-polymer which is made by linking a polymer or polymers of one type to another polymer molecule of a different composition.

The term "terpolymer," as used herein, refers to a co-polymer comprising three different monomers.

The term "bio-polymer," as used herein, refers to a naturally occurring polymer. Naturally occurring polymers include, but are not limited to, proteins and carbohydrates. The term "bio-polymer" also includes derivatised forms of the naturally occurring polymers that have been modified to facilitate cross-linking to a synthetic polymer of the invention.

The term "synthetic polymer," as used herein, refers to a polymer that is not naturally occurring and that is produced by chemical or recombinant synthesis.

The terms "alkyl" and "lower alkyl" are used interchangeably herein to refer to a straight chain or branched alkyl group of one to eight carbon atoms or a cycloalkyl group of three to eight carbon atoms. These terms are further exemplified by such groups as methyl, ethyl, *n*-propyl, *i*-propyl, *n*-butyl, *t*-butyl, l-butyl (or 2-methylpropyl), *i*-amyl, *n*-amyl, hexyl, cyclopropyl, cyclobutyl, cyclopentyl, cyclohexyl and the like.

The term "bioactive agent," as used herein, refers to a molecule or compound which exerts a physiological, therapeutic or diagnostic effect *in vivo.* Bioactive agents may be organic or inorganic. Representative examples include proteins, peptides, carbohydrates, nucleic acids and fragments thereof, anti-tumour and anti-neoplastic compounds, anti-viral compounds, anti-inflammatory compounds, antibiotic compounds such as antifungal and antibacterial compounds, cholesterol lowering drugs, analgesics, contrast agents for medical diagnostic imaging, enzymes, cytokines, local anaesthetics, hormones, anti-angiogenic agents, neurotransmitters, therapeutic oligonucleotides, viral particles, vectors, growth factors, retinoids, cell adhesion factors, extracellular matrix glycoproteins (such as laminin), hormones, osteogenic factors, antibodies and antigens.

The term "biocompatible," as used herein, refers to an ability to be incorporated into a biological system, such as into an organ or tissue of an animal, without stimulating an immune and / or inflammatory response, fibrosis or other adverse tissue response.

As used herein, the term "about" refers to a +/-10% variation from the nominal value. It is to be understood that such a variation is always included in any given value provided herein, whether or not it is specifically referred to.

### 1. BIO-SYNTHETIC MATRIX

The present invention provides a bio-synthetic matrix comprising a hydrogel which is formed by cross-linking a synthetic polymer and a bio-polymer. The bio-polymer may be in its naturally-occurring form, or it may be derivatised to facilitate cross-linking to the synthetic polymer. The matrix is robust, biocompatible and non-cytotoxic. The matrix can be formed in aqueous solution at neutral pH and can be tailored to further comprise one or more bioactive agents such as growth factors, retinoids, cell adhesion factors, enzymes, peptides, proteins, nucleotides, drugs, and the like. The bioactive agent can be covalently attached to the synthetic polymer, or it may be encapsulated and dispersed within the final matrix depending on the end use demands for the matrix. The matrix may also comprise cells encapsulated and dispersed therein, which are capable of proliferation and/or diversification upon deposition of the matrix *in vivo.*

In one embodiment of the present invention, the bio-synthetic matrix supports cell growth. Such cell growth may be epithelial and/or endothelial surface coverage (*i.e.* two dimensional, 2D, growth) and/or three-dimensional (3D) cell growth involving growth into the matrix itself.

In another embodiment of the invention, the bio-synthetic matrix supports nerve in-growth. As is known in the art, nerve growth into transplanted tissue takes place over an extended period of time, typically in the order of months or years. Growth of nerves into the matrix can occur more rapidly than growth of nerves into transplanted tissue thus leading to more rapid regeneration of functional tissue, for example, nerve in-growth may occur within weeks.

The bio-synthetic matrix can be tailored for specific applications. For example, the matrix can be used in tissue engineering applications and may be pre-formed into a specific shape for this purpose. The matrix can also be used as a drug delivery vehicle to provide sustained release of a therapeutic or diagnostic compound at a particular site within the body of an animal.

In order to be suitable for *in vivo* implantation for tissue engineering purposes, the bio-synthetic matrix must maintain its form at physiological temperatures, be substantially insoluble in water, be adequately robust, and support the growth of cells. It may also be desirable for the matrix to support the growth of nerves.

### 1.1 Synthetic Polymer

In accordance with the present invention, the synthetic polymer that is incorporated into the bio-synthetic matrix is a co-polymer comprising one or more acrylamide derivatives, one or more hydrophilic co-monomers and one or more derivatised carboxylic acid co-monomers which comprise pendant cross-linkable moieties.

As used herein, an "acrylamide derivative" refers to a N-alkyl or N,N-dialkyl substituted acrylamide or methacrylamide. Examples of acrylamide derivatives suitable for use in the synthetic polymer of the present invention include, but are not limited to, N-methylacrylamide, N-ethylacrylamide, N-isopropylacrylamide (NiPAAm), N-octylacrylamide, N-cyclohexylacrylamide, N-methyl-N-ethylacrylamide, N-methylmethacrylamide, N-ethylmethacrylamide, N-isopropylmethacrylamide, N,N-dimethylacrylamide, N,N-diethylacrylamide, N,N-dimethylmethacrylamide, N,N-diethylmethacrylamide, N,N-dicyclohexylacrylamide, N-methyl-N-cyclohexylacrylamide, N-acryloylpyrrolidine, N-vinyl-2-pyrrollidinone, N-methacryloylpyrrolidine, and combinations thereof.

A "hydrophilic co-monomer" in the context of the present invention is a hydrophilic monomer that is capable of co-polymerisation with the acrylamide derivative and the derivatised carboxylic acid components of the synthetic polymer. The hydrophilic co-monomer is selected to provide adequate solubility for polymerisation, aqueous solubility of the co-polymer and freedom from phase transition of the final co-polymer and hydrogel. Examples of suitable hydrophilic co-monomers are hydrophilic acryl- or methacryl- compounds such as carboxylic acids including acrylic acid, methacrylic acid and derivatives thereof, acrylamide, methacrylamide, hydrophilic acrylamide derivatives, hydrophilic methacrylamide derivatives, hydrophilic acrylic acid esters, hydrophilic methacrylic acid esters, vinyl ethanol and its derivatives and ethylene glycols. The carboxylic acids and derivatives may be, for example, acrylic acid, methacrylic acid, 2-hydroxyethyl methacrylate (HEMA), or a combination thereof. Examples of hydrophilic acrylamide derivatives include, but are not limited to, N,N-dimethylacrylamide, N,N-diethylacrylamide, 2-[N,N-dimethylamino]ethylacrylamide, 2-[N,N-diethylamino]ethylacrylamide, N,N-diethylmethacrylamide, 2-[N,N-dimethylamino] ethylmethacrylamide, 2-[N,N-diethylamino]ethylmethacrylamide, N-vinyl-2-pyrrollidinone, or combinations thereof. Examples of hydrophilic acrylic esters include, but are not limited to, 2-[N,N-diethylamino] ethylacrylate, 2-[N,N-dimethylamino]ethylacrylate, 2-[N,N-diethylamino]ethylmethacrylate, 2-[N,N-dimethylamino]ethylmethacrylate, or combinations thereof.

As used herein, a "derivatised carboxylic acid co-monomer" refers to a hydrophilic acryl- or methacryl- carboxylic acid, for example, acrylic acid, methacrylic acid, or a substituted version thereof, which has been chemically derivatised to contain one or more cross-linking moieties, such as succinimidyl groups, imidazoles, benzotriazoles and p-nitrophenols. The term "succinimidyl group" is intended to encompass variations of the generic succinimidyl group, such as sulphosuccinimidyl groups. Other similar structures such as 2-(N-morpholino)ethanesulphonic acid will also be apparent to those skilled in the art. In the context of the present invention the group selected as a cross-linking moiety acts to increase the reactivity of the carboxylic acid group to which it is attached towards primary amines (*i.e.* -NH₂ groups) and thiols (*i.e.* -SH groups). Examples of suitable groups for derivatisation of the carboxylic acid co-monomers for use in the synthetic polymer include, but are not limited to, N-succinimide, N-succinimide-3-sulphonic acid, N-benzotriazole, N-imidazole and *p-*nitrophenol.

In one embodiment of the present invention, the synthetic polymer comprises:
(a) one or more acrylamide derivative of general formula I: 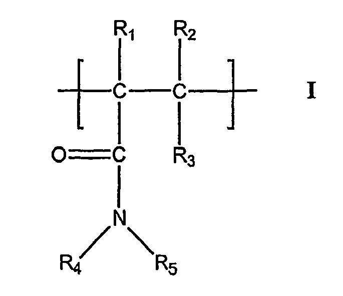 wherein:
   R₁, R₂, R₃, R₄ and R₅ are independently selected from the group of: H and lower alkyl;
(b) one or more hydrophilic co-monomer (which may be the same or different to (a)) having the general formula II: 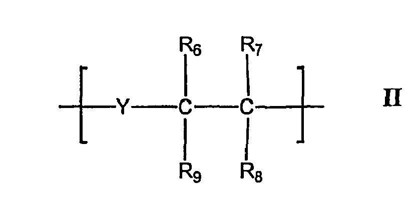 wherein:
   Y is O or is absent;
   R₆, and R₇ are independently selected from the group of: H and lower alkyl;
   R₈ is H, lower alkyl, or-OR', where R' is H or lower alkyl; and
   R₉ is H, lower alkyl, or -C(O)R₁₀, and
   R₁₀ is -NR₄R₅ or -OR", where R" is H or CH₂CH₂OH;
and (c) one or more derivatised carboxylic acid having the general formula III: 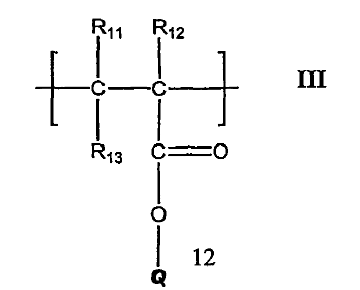
wherein:
R₁₁, R₁₂ and R₁₃ are independently selected from the group of: H and lower alkyl and
Q is N-succinimido, 3-sulpho-succinimdo (sodium salt), N-benzotriazolyl, N-imidazolyl or *p*-nitrophenyl.

In one embodiment, the synthetic polymer comprises one or more acrylamide derivative of general formula I, one or more hydrophilic co-monomer of general formula II and one or more derivatised carboxylic acid of general formula III, as described above, wherein the term "lower alkyl" refers to a branched or straight chain alkyl group having 1 to 8 carbon atoms.

In another embodiment, the synthetic polymer comprises one or more acrylamide derivative of general formula I, one or more hydrophilic co-monomer of general formula II and one or more derivatised carboxylic acid of general formula III, as described above, wherein the term "lower alkyl" refers to to a cycloalkyl group having 3 to 8 carbon atoms, such as cyclopropyl, cyclobutyl, cyclopentyl and cyclohexyl.

The co-polymer may be linear or branched, regular, random or block. In accordance with the present invention, the final synthetic polymer comprises a plurality of pendant reactive moieties available for cross-linking, or grafting, of appropriate biomolecules.

A worker skilled in the art will appreciate that the group of compounds encompassed by the term "acrylamide derivative" and the group of compounds encompassed by the term "hydrophilic co-monomer" overlap substantially and that a single monomer could be selected that fulfils the functions of both these components in the co-polymer. Thus, for example, when an acrylamide derivative is selected that is sufficiently hydrophilic to confer on the synthetic polymer the desired properties, then a hydrophilic co-monomer component may be chosen that is identical to the selected acrylamide derivative resulting in a co-polymer that comprises two different monomers only (*i.e.* the acrylamide derivative/hydrophilic co-monomer and the derivatised carboxylic acid co-monomer). On the other hand, when enhanced hydrophilicity beyond that provided by the selected acrylamide derivative is desired, then one or more different hydrophilic co-monomers may be chosen resulting in a co-polymer comprising at least three different monomers.

In one embodiment of the present invention, the acrylamide derivative and the hydrophilic co-monomer used in the preparation of the synthetic polymer are the same. In another embodiment, the acrylamide derivative and the hydrophilic co-monomer used in the preparation of the synthetic polymer are different.

The overall hydrophilicity of the co-polymer is controlled to confer water solubility at 0°C to physiological temperatures without precipitation or phase transition. In one embodiment of the present invention, the co-polymer is water soluble between about 0°C and about 37°C.

The co-polymer should be sufficiently soluble in aqueous solution to facilitate hydrogel formation. In accordance with one embodiment of the present invention, therefore, the term "water soluble" is intended to refer to an aqueous solubility of the co-polymer of at least about 0.5 weight/volume (w/v) %. In another embodiment, the co-polymer has an aqueous solubility of between about 1.0 w/v % and about 50 w/v %. In a further embodiments, the co-polymer has an aqueous solubility of about 5 w/v % and about 45 w/v % and between about 10% w/v and about 35% w/v.

As is known in the art, most synthetic polymers have a distribution of molecular mass and various different averages of the molecular mass are often distinguished, for example, the number average molecular mass (Mₙ) and the weight average molecular mass (M_{w}). The molecular weight of a synthetic polymer is usually defined in terms of its number average molecular mass (Mₙ), which in turn is defined as the sum of nᵢMᵢ divided by the sum of nᵢ ,where nᵢ is the number of molecules in the distribution with mass Mᵢ. The synthetic polymer for use in the matrix of the present invention typically has a number average molecular mass (Mₙ) between 2,000 and 1,000,000. In one embodiment of the present invention, the Mₙ of the polymer is between about 5,000 and about 90,000. In another embodiment of the present invention, the Mₙ of the polymer is between about 25,000 and about 80,000. In a further embodiment, the Mₙ of the polymer is between about 30,000 and about 50,000. In another embodiment, the Mₙ of the polymer is between about 50,000 and about 60,000.

It is also well-known in the art that certain water-soluble polymers exhibit a lower critical solution temperature (LCST) or "cloud point." The LCST of a polymer is the temperature at which phase separation occurs (*i.e.* the polymer begins to separate from the surrounding aqueous medium). Typically, for those polymers or hydrogels that are clear, the LCST also corresponds to the point at which clarity begins to be lost. It will be readily apparent that for certain tissue engineering applications, the presence or absence of phase separation in the final hydrogel may not be relevant provided that the hydrogel still supports cell growth. For other applications, however, a lack of phase separation in the final hydrogel may be critical. For example, for optical applications, clarity (and, therefore, the absence of any phase transition) will be important.

Thus, in accordance with one embodiment of the present invention, co-polymers with a LCST between about 35°C and about 60°C are selected for use in the hydrogels. In another embodiment, co-polymers with a LCST between about 42°C and about 60°C are selected for use in the hydrogels It is also known in the art that the LCST of a polymer may be affected by the presence of various solutes, such as ions or proteins, and by the nature of compounds cross-linked or attached to the polymer. Such effects can be determined empirically using standard techniques and selection of a synthetic polymer with an appropriate LCST for a particular application is considered to be within the ordinary skills of a worker in the art.

In order for the synthetic polymer to be suitably robust and thermostable, it is important that the ratio of acrylamide derivative(s) to hydrophilic co-monomer(s) is optimised when different monomers are used for these components. Accordingly, the acrylamide derivative(s) are present in the synthetic polymer in the highest molar ratio. In addition, the number of derivatised carboxylic acid co-monomer(s) present in the final polymer will determine the ability of the synthetic gel to form cross-links with the bio-polymer in the bio-synthetic matrix. Selection of suitable molar ratios of each component to provide a final synthetic polymer with the desired properties is within the ordinary skills of a worker in the art.

In accordance with one embodiment of the present invention, when different monomers are being used as the acrylamide derivative and hydrophilic co-monomer components, the amount of acrylamide derivative in the polymer is between 50% and 90%, the amount of hydrophilic co-monomer is between 5% and 50%, and the amount of derivatised carboxylic acid co-monomer is between 0.1% and 15%, wherein the sum of the amounts of acrylamide derivative, hydrophilic co-monomer and derivatised carboxylic acid co-monomer is 100%, wherein the % value represents the molar ratio.

In accordance with another embodiment of the invention, the synthetic polymer is prepared using the same monomer as both the acrylamide derivative and the hydrophilic co-monomer and the molar ratio of the acrylamide derivative/hydrophilic co-monomer is between about 50% and about 99.5% and the molar ratio of the derivatised carboxylic acid co-monomer is between about 0.5% and about 50%.

In accordance with a further embodiment, the combined molar ratio of the acrylamide derivative and the hydrophilic co-monomer is between about 80% and about 99% and the molar ratio of the derivatised carboxylic acid co-monomer is between about 1% and about 20%.

One skilled in the art will appreciate that the selection and ratio of the components in the synthetic polymer will be dependent to varying degrees on the final application for the bio-synthetic matrix. For example, for ophthalmic applications, it is important that the final matrix be clear, whereas for other tissue engineering applications, the clarity of the matrix may not be an important factor.

In one embodiment of the present invention, the synthetic polymer is a random or block co-polymer comprising one acrylamide derivative, one hydrophilic co-monomer and one derivatised carboxylic acid co-monomer (a "terpolymer"). In another embodiment, the synthetic polymer is a terpolymer comprising NiPAAm monomer, acrylic acid (AAc) monomer and a derivatised acrylic acid monomer. In a further embodiment, the synthetic polymer is a terpolymer comprising NiPAAm monomer, acrylamide (AAm) monomer and derivatised acrylic acid monomer. In another embodiment, the derivatised acrylic acid monomer is N-acryloxysuccinimide. In another embodiment, a terpolymer is prepared with a feed ratio that comprises NiPAAm monomer, AAc monomer and N-acryloxysuccinimide in a ratio of about 85:10:5 molar %.

In an alternate embodiment of the invention, the synthetic polymer is a random or block co-polymer comprising one acrylamide derivative/hydrophilic co-monomer and one carboxylic acid co-monomer. In another embodiment, the synthetic polymer comprises DMAA monomer and a derivatised acrylic acid monomer. In a further embodiment, the derivatised acrylic acid monomer is N-acryloxysuccinimide. In another embodiment, a synthetic polymer is prepared with a feed ratio that comprises DMAA monomer and N-acryloxysuccinimide in a ratio of about 95:5 molar %.

### 1.2 Bio-polymer

Bio-polymers are naturally-occurring polymers, such as proteins and carbohydrates. In accordance with the present invention, the bio-synthetic matrix comprises a bio-polymer or a derivatised version thereof cross-linked to the synthetic polymer by means of the pendant cross-linking moieties in the latter. Thus, for the purposes of the present invention the bio-polymer contains one or more groups which are capable of reacting with the cross-linking moiety (*e.g.* a primary amine or a thiol), or can be derivatised to contain such a group. Examples of suitable bio-polymers for use in the present invention include, but are not limited to, collagens (including Types I, II, III, IV and V), denatured collagens (or gelatins), recombinant collagens, fibrin-fibrinogen, elastin, glycoproteins, alginate, chitosan, hyaluronic acid, chondroitin sulphates and glycosarninoglycans (or proteoglycans). One skilled in the art will appreciate that some of these bio-polymers may need to be derivatised in order to contain a suitable reactive group as indicated above, for example, glucosaminoglycans need to be deacetylated or desulphated in order to possess a primary amine group. Such derivatisation can be achieved by standard techniques and is considered to be within the ordinary skills of a worker in the art.

Suitable bio-polymers for use in the invention can be purchased from various commercial sources or can be prepared from natural sources by standard techniques.

### 1.3 Bioactive Agents

As indicated above, the synthetic polymer to be included in the bio-synthetic matrix the present invention contains a plurality of pendant cross-linking moieties. It will be apparent that sufficient cross-linking of the synthetic and bio-polymers to achieve a suitably robust matrix can be achieved without reaction of all free cross-linking groups. Excess groups may, therefore, optionally be used to covalently attach desirable bioactive agents to the matrix. Non-limiting examples of bioactive agents that may be incorporated into the matrix by cross-linking include, for example, growth factors, retinoids, enzymes, cell adhesion factors, extracellular matrix glycoproteins (such as laminin, fibronectin, tenascin and the like), hormones, osteogenic factors, cytokines, antibodies, antigens, and other biologically active proteins, certain pharmaceutical compounds, as well as peptides, fragments or motifs derived from biologically active proteins.

In one embodiment of the present invention, the cross-linking groups are succinimidyl groups and suitable bioactive agents for grafting to the polymer are those which contain either primary amino or thiol groups, or which can be readily derivatised so as to contain these groups.

### 2. METHOD OF PREPARING THE BIO-SYNTHETIC MATRIX

### 2.1 Preparation of the Synthetic Polymer

Co-polymerization of the components for the synthetic polymer can be achieved using standard methods known in the art [for example, see A. Ravve "Principles of Polymer Chemistry", Chapter 3. Plenum Press, New York 1995]. Typically appropriate quantities of each of the monomers are dispersed in a suitable solvent in the presence of an initiator. The mixture is maintained at an appropriate temperature and the co-polymerisation reaction is allowed to proceed for a pre-determined period of time. The resulting polymer can then be purified from the mixture by conventional methods, for example, by precipitation.

The solvent for the co-polymerisation reaction may be a non-aqueous solvent if one or more monomer is sensitive to hydrolysis or it may be an aqueous solvent. Suitable aqueous solvents include, but are not limited to, water, buffers and salt solutions. Suitable non-aqueous solvents are typically cyclic ethers (such as dioxane), chlorinated hydrocarbons (for example, chloroform) or aromatic hydrocarbons (for example, benzene). The solvent may be nitrogen purged prior to use, if desired. In one embodiment of the present invention, the solvent is a non-aqueous solvent. In another embodiment, the solvent is dioxane.

Suitable polymerisation initiators are known in the art and are usually free-radical initiators. Examples of suitable initiators include, but are not limited to, 2,2'-azobisisobutyronitrile (AIBN), other azo compounds, such as 2,2'-azobis-2-ethylpropionitrile; 2,2'-azobis-2-cyclopropylpropionitrile; 2,2'-azobiscyclohexanenitrile; 2,2'-azobiscyclooctanenitrile, and peroxide compounds, such as dibenzoyl peroxide and its substituted analogues, and persulfates, such as sodium, potassium, and the like.

Once the polymer has been prepared, and purified if necessary, it can be characterised by various standard techniques. For example, the molar ratio composition of the polymer can be determined by nuclear magnetic resonance spectroscopy (proton and/or carbon-13) and bond structure can be determined by infrared spectroscopy. Molecular mass can be determined by gel permeation chromatography and/or high pressure liquid chromatography. Thermal characterisation of the polymer can also be conducted, if desired, for example by determination of the melting point and glass transition temperatures using differential scanning calorimetric analysis. Aqueous solution properties such as micelle and gel formation, and LCST can be determined using visual observation, fluorescence spectroscopy, UV-visible spectroscopy and laser light scattering instruments.

In one embodiment of the present invention, the synthetic polymer is prepared by dispersing the monomers in nitrogen-purged dioxane in the presence of the initiator AIBN and allowing polymerisation to proceed at a temperature of about 60°C to 70°C. The resulting polymer is purified by repeated precipitation.

### 2.2 Preparation of the Hydrogel

Cross-linking of the synthetic polymer and bio-polymer can be readily achieved by mixing appropriate amounts of each polymer at room temperature in a suitable solvent. Typically the solvent is an aqueous solvent, such as a salt solution, buffer solution, cell culture medium, or a diluted or modified version thereof. One skilled in the art will appreciate that in order to preserve triple helix structure of polymers such as collagen and to prevent fibrillogenisis and/or opacification of the hydrogel, the cross-linking reaction should be conducted in aqueous media with close control of the pH and temperature. The significant levels of amino acids in nutrient media normally used for cell culture can cause side reactions with the cross-linking moieties of the synthetic polymer, which can result in diversion of these groups from the cross-linking reaction. Use of a medium free of amino acids and other proteinaceous materials can help to prevent these side reactions and, therefore, increase the number of cross-links that form between the synthetic and bio-polymers. Conducting the cross-linking reaction in aqueous solution at room or physiological temperatures allows both cross-linking and the much slower hydrolysis of any residual cross-linking groups to take place.

Alternatively, a termination step can be included to react any residual cross-liking groups in the matrix. For example, one or more wash steps in a suitable buffer containing glycine will terminate any residual cross-linking groups as well as removing any side products generated during the cross-linking reaction. Unreacted cross-linking groups may also be terminated with a polyfunctional amine such as lysine or triethylenetetraamine leading to formation of additional short, inter-chain cross-links. Wash steps using buffer alone can also be conducted if desired in order to remove any side products from the cross-linking reaction. If necessary, after the cross-linking step, the temperature of the cross-linked polymer suspension can be raised to allow the hydrogel to form fully.

In accordance with the present invention, the components of the hydrogel are chemically cross-linked so as to be substantially non-extractable, i.e. the bio-polymer and synthetic polymer do not exude extensively from the gel under physiological conditions. In accordance with one embodiment of the present invention, the amount of bio-polymer or synthetic polymer that can be extracted from the matrix into aqueous media under physiological conditions over a period of 24 hours is less than 5% by weight of either component. In another embodiment, the amount of bio-polymer or synthetic polymer that can be extracted from the matrix into aqueous media under physiological conditions over a period of 24 hours is less than 2% by weight of either component. In further embodiments, the amount that can be extracted over a period of 24 hours is less than 1% by weight, less than 0.5% by weight and less than 0.2% by weight.

The amount of bio-polymer and/or synthetic polymer that can be extracted from the matrix into aqueous media can be determined *in vitro* using standard techniques (for example, the USP Basket Method). Typically, the matrix is placed in an aqueous solution of a predetermined pH, for example around pH 7.4 to simulate physiological conditions. The suspension may or may not be stirred. Samples of the aqueous solution are removed at predetermined time intervals and are assayed for polymer content by standard analytical techniques.

One skilled in the art will understand that the amount of each polymer to be included in the hydrogel will be dependent on the choice of polymers and the intended application for the hydrogel. In general, using higher initial amounts of each polymer will result in the formation of a more robust gel due to the lower water content and the presence of a greater amount of cross-linked polymer. Higher quantities of water or aqueous solvent will produce a soft hydrogel. In accordance with the present invention, the final hydrogel comprises between about 20 and 99.6 % by weight of water or aqueous solvent, between about 0.1 and 30 % by weight of synthetic polymer and between about 0.3 and 50 % by weight of bio-polymer.

In one embodiment of the present invention, the final hydrogel comprises between about 40 and 99.6 % by weight of water or aqueous solvent, between about 0.1 and 30 % by weight of synthetic polymer and between about 0.3 and 30 % by weight of bio-polymer. In another embodiment, the final hydrogel comprises between about 60 and 99.6 % by weight of water or aqueous solvent, between about 0.1 and 10 % by weight of synthetic polymer and between about 0.3 and 30 % by weight of bio-polymer. In a further embodiment, the final hydrogel comprises between about 80 and 98.5 % by weight of water or aqueous solvent, between about 0.5 and 5 % by weight of synthetic polymer and between about 1 and 15 % by weight of bio-polymer. In other embodiments, the final hydrogel contains about 95 to 97 % by weight of water or aqueous solvent and between about 1- 2 % by weight of synthetic polymer and about 2 - 3 % by weight of bio-polymer; and about 94 to 98 % by weight of water or aqueous solvent and between about 1 - 3 % by weight of synthetic polymer and about 1 - 3 % by weight of bio-polymer.

Similarly, the relative amounts of each polymer to be included in the hydrogel will be dependent on the type of synthetic polymer and bio-polymer being used and upon the intended application for the hydrogel. One skilled in the art will appreciate that the relative amounts bio-polymer and synthetic polymer will influence the final gel properties in various ways, for example, high quantities of bio-polymer will produce a very stiff hydrogel. One skilled in the art will appreciate that the relative amounts of each polymer in the final matrix can be described in terms of the weight: weight ratio of the bio-polymer : synthetic polymer or in terms of equivalents of reactive groups. In accordance with the present invention, the weight per weight ratio of bio-polymer : synthetic polymer is between about 1 : 0.07 and about 1 : 14. In one embodiment, the w/w ratio of bio-polymer : synthetic polymer is between 1 : 1.3 and 1 : 7. In another embodiment, the w/w ratio of bio-polymer : synthetic polymer is between 1 : 1 and 1 : 3. In a further embodiment, the w/w ratio of bio-polymer : synthetic polymer is between 1 : 0.7 and 1 : 2.

In an alternative embodiment of the present invention, the matrix comprises a proteinaceous bio-polymer and a synthetic polymer comprising pendant N-acryloxysuccinimide groups. In this embodiment of the invention, the ratio of bio-polymer : synthetic polymer is described in terms of molar equivalents of free amine groups in the bio-polymer to N-acryloxysuccinimide groups and is between 1 : 0.5 and 1: 20. In another embodiment, this ratio is between 1 : 1.8 and 1 : 10. In a further embodiments, the ratio is between 1 : 1 and 1 : 5, and between 1 : 1 and 1 : 3.

### 2.3 Incorporation of Bioactive Agents into the Bio-synthetic Matrix

Bioactive agents can be incorporated into the matrix if desired either by covalent attachment (or "grafting") to the synthetic polymer through the pendant cross-linking moieties, or by encapsulation within the matrix. Examples of bioactive agents that may be covalently attached to the synthetic polymer component of the matrix are provided above. If necessary, the bioactive agent may be first derivatised by standard procedures to provide appropriate reactive groups for reaction with the cross-linking groups. For covalent attachment of a bioactive agent, the synthetic polymer is first reacted with the bioactive agent and then this modified synthetic polymer subsequently cross-linked to the bio-polymer as described above. Reaction of the bioactive agent with the synthetic polymer can be conducted under standard conditions, for example by mixing the bioactive agent and the synthetic polymer together in a non-aqueous solvent, such as N,N-dimethyl formamide, dioxane, dimethyl sulphoxide and N,N-dimethylacrylamide. The use of a non-aqueous solvent avoids hydrolysis of the reactive groups during incorporation of the bioactive agent. Alternatively, the reaction may be conducted in an aqueous solvent as described above for the cross-linking reaction.

Bioactive agents which are not suitable for grafting to the polymer, for example, those that do not contain primary amino or free thiol groups for reaction with the cross-linking groups in the synthetic polymer, or which cannot be derivatised to provide such groups, can be entrapped in the final matrix. Examples of bioactive agents which may be entrapped in the matrix include, but are not limited to, pharmaceutical drugs, diagnostic agents, viral vectors, nucleic acids and the like. For entrapment, the bioactive agent is added to a solution of the synthetic polymer in an appropriate solvent prior to mixture of the synthetic polymer and the bio-polymer to form a cross-linked hydrogel. Alternatively, the bioactive agent can be added to a solution containing both the synthetic and bio-polymers prior to the cross-linking step. The bioactive agent is mixed into the polymer solution such that it is substantially uniformly dispersed therein, and the hydrogel is subsequently formed as described above. Appropriate solvents for use with the bioactive agent will be dependent on the properties of the agent and can be readily determined by one skilled in the art.

### 2.4 Entrapment of Cells in the Bio-synthetic Matrix

The bio-synthetic matrix according to the present invention may also comprise cells entrapped therein and thus permit delivery of the cells to a tissue or organ *in vivo.* A variety of different cell types may be delivered using the bio-synthetic matrix, for example, myocytes, ocular cells (*e.g.* from the different corneal layers), adipocytes, fibromyoblasts, ectodermal cells, muscle cells, osteoblasts (*i.e.* bone cells), chondrocytes (*i.e.* cartilage cells), endothelial cells, fibroblasts, pancreatic cells, hepatocytes, bile duct cells, bone marrow cells, neural cells, genitourinary cells (including nephritic cells), or combinations thereof. The matrix may also be used to deliver totipotent stem cells, pluripotent or committed progenitor cells or reprogrammed (dedifferentiated) cells to an *in vivo* site such that cells of the same type as the tissue can be produced. For example, mesenchymal stem cells, which are undifferentiated, can be delivered in the matrix. Examples of mesenchymal stem cells include those which can diversify to produce osteoblasts (to generate new bone tissue), chondrocytes (to generate new cartilaginous tissue), and fibroblasts (to produce new connective tissue). Alternatively, committed progenitor cells capable of proliferating to provide cells of the same type as those present at the *in vivo* site can be used, for example, myoblasts, osteoblasts, fibroblasts and the like.

Cells can be readily entrapped in the final matrix by addition of the cells to a solution of the synthetic polymer prior to admixture with the bio-polymer to form a cross-linked hydrogel. Alternatively, the cells can be added to a solution containing both the synthetic and bio-polymers prior to the cross-linking step. The synthetic polymer may be reacted with a bioactive agent prior to admixture with the cells if desired. Typically, for the encapsulation of cells in the matrix, the various components (cells, synthetic polymer and bio-polymer) are dispersed in an aqueous medium, such as a cell culture medium or a diluted or modified version thereof. The cell suspension is mixed gently into the polymer solution until the cells are substantially uniformly dispersed in the solution, then the hydrogel is formed as described above.

### 2.5 Other Elements

The present invention also contemplates the optional inclusion of one or more reinforcing material in the bio-synthetic matrix to improve the mechanical properties of the matrix such as the strength, resilience, flexibility and/or tear resistance. Thus, the matrix may be reinforced with flexible or rigid fibres, fibre mesh, fibre cloth and the like. The use of such reinforcing materials is known in the art, for example, the use of fibres, cloth, or sheets made from collagen fibrils, oxidised cellulose or polymers such as polylactic acid, polyglycolic acid or polytetrafluoroethylene in implantable medical applications is known.

The reinforcing material can be incorporated into the matrix using standard protocols. For example, an aqueous solution of synthetic and bio-polymers in an appropriate buffer can be added to a fibre cloth or mesh, such as Interceed (Ethicon Inc., New Brunswick, N.J.). The aqueous solution will flow into the interstices of the cloth or mesh prior to undergoing cross-linking and will thus form a hydrogel with the cloth or mesh embedded therein. Appropriate moulds can be used to ensure that the fibres or fibre mesh are contained entirely within the hydrogel if desired. The composite structure can subsequently be washed to remove any side products generated during the cross-linking reaction. Typically, the fibres used are hydrophilic in nature to ensure complete wetting by the aqueous solution of polymers.

One skilled in the art will appreciate that, for applications requiring high optical clarity, the structure of the reinforcement should be selected to prevent light scattering from the final composite matrix, for example, by the use of nano-fibers and/or careful refractive index matching of reinforcement and hydrogel.

### 3. TESTING THE BIO-SYNTHETIC MATRIX

In accordance with the present invention, the bio-synthetic matrix comprises a hydrogel with or without added bioactive agents and/or encapsulated cells. In order to be suitable for *in vivo* implantation for tissue engineering purposes, the bio-synthetic matrix must maintain its form at physiological temperatures, be adequately robust, be substantially insoluble in water, and support the growth of cells. It may also be desirable for the matrix to support the growth of nerves. It will be readily appreciated that for certain specialised applications, the matrix may require other characteristics. For example, for surgical purposes, the matrix may need to be relatively flexible as well as strong enough to support surgical manipulation with suture thread and needle, and for ophthalmic applications, such as cornea repair or replacement, the optical clarity of the matrix will be important.

### 3.1 Physical / Chemical testing

When used for tissue engineering applications, the bio-synthetic matrix needs to meet the mechanical parameters necessary to prevent the matrix tearing or rupturing when submitted to surgical procedures and to provide adequate support for cell growth once in place. The ability of matrix to resist tearing is related to its intrinsic mechanical strength, the form and thickness of the matrix and the tension being applied.

The ability of the bio-synthetic matrix to withstand shearing forces, or tearing can be roughly determined by applying forces in opposite directions to the specimen using two pairs of forceps. Alternatively, a suitable apparatus can be used to measure quantitatively the ability of the matrix to withstand shearing forces. Tensiometers for this purpose are available commercially, for example, from MTS, Instron, and Cole Parmer.

For testing, the matrix can be formed into sheets and then cut into appropriately sized strips. Alternatively, the matrix can be moulded into the desired shape for tissue engineering purposes and the entire moulded matrix can be tested. To calculate tensile strength, the force at rupture, or "failure," of the matrix is divided by the cross-sectional area of the test sample, resulting in a value that can be expressed in force (N) per unit area. The stiffness (modulus) of the matrix is calculated from the slope of the linear portion of the stress/strain curve. Strain is the real-time change in length during the test divided by the initial length of the test sample before the test begins. The strength at rupture is the final length of the test sample when it ruptures minus the length of the initial test sample, divided by this initial length.

One skilled in the art will appreciate that because of the softness of hydrogels and exudation of the aqueous component when clamped, meaningful tensile data can be difficult to obtain from hydrogels. Quantitative characterisation of tensile strength in hydrogels can be achieved, for example, through the use of suture pull-out measurements on moulded matrix samples. Typically, a suture is placed about 2 mm from the edge of a test sample and the peak force that needs to be applied in order to rip the suture through the sample is measured. For example, for a test sample of matrix intended for ophthalmic applications that has been moulded in the shape and thickness of a human cornea, two diametrically opposed sutures can be inserted into the matrix, as would be required for the first step in ocular implantation. The two sutures can then be pulled apart at about 10 mm/min on a suitable instrument, such as an Instron Tensile Tester. Strength at rupture of the matrix is calculated, together with elongation at break and elastic modulus [see, for example, Zeng et al., J. Biomech., 34:533-537 (2001)]. It will be appreciated by those skilled in the art that, for those bio-synthetic matrices intended for surgical applications, the matrices need not be as strong (*i.e.* have the same ability to resist tearing) as mammalian tissue. The determining factor for the strength of the matrix in such applications is whether or not it can be sutured in place by a careful and experienced surgeon.

If desired, the LCST of the bio-synthetic hydrogel matrix can be measured using standard techniques. For example, LCST can be calculated by heating samples of the matrix at about 0.2°C per minute and visually observing the cloud point (see, for example, H. Uludag, et al., J. Appl. Polym. Sci. 75:583 - 592 (2000)).

Permeability of the bio-synthetic matrix can be determined by assessing the glucose permability coefficient and/or the average pore sizes for the matrix using standard techniques such as PBS permeability assessment using a permeability cell and/or atomic force microscopy. In accordance with one embodiment of the present invention, the bio-synthetic matrix has an average pore size between about 90 nm and about 500 nm. In another embodiment, the matrix has an average pore size between about 100 nm and about 300 nm.

Optical transmission and light scatter can also be measured for matrices intended for ophthalmic applications using a custom-built instrument that measures both transmission and scatter [see, for example, Priest and Munger, Invest. Ophthalmol. Vis. Sci. 39: S352 (1998)].

### 3.2 In vitro Testing

It will be readily appreciated that the bio-synthetic matrix must be non-cytotoxic and biocompatible in order to be suitable for *in vivo* use. The cytotoxicity of the bio-synthetic matrix can be assessed using standard techniques such as the Ames assay to screen for mutagenic activity, the mouse lymphoma assay to screen for the ability of the matrix to induce gene mutation in a mammalian cell line, *in vitro* chromosomal aberration assays using, for example, Chinese hamster ovary cells (CHO) to screen for any DNA rearrangements or damage induced by the matrix. Other assays include the sister chromatid assay, which determines any exchange between the arms of a chromosome induced by the matrix and *in vitro* mouse micronucleus assays to determine any damage to chromosomes or to the mitotic spindle. Protocols for these and other standard assays are known in the art, for example, see *OECD Guidelines for the Testing of Chemicals* and protocols developed by the ISO.

The ability of the matrix to support cell growth can also be assessed *in vitro* using standard techniques. For example, cells from an appropriate cell line, such as human epithelial cells, can be seeded either directly onto the matrix or onto an appropriate material surrounding the matrix. After growth in the presence of a suitable culture medium for an appropriate length of time, confocal microscopy and histological examination of the matrix can be conducted to determine whether the cells have grown over the surface of and/or into the matrix.

The ability of the matrix to support in-growth of nerve cells can also be assessed *in vitro.* For example, a nerve source, such as dorsal root ganglia, can be embedded into an appropriate material surrounding the matrix or directly inserted into the matrix. An example of a suitable material would be a soft collagen based gel. Cells from an appropriate cell line can then be seeded either directly onto the matrix or onto an appropriate material surrounding the matrix and the matrix can be incubated in the presence of a suitable culture medium for a pre-determined length of time. Examination of the matrix, directly and / or in the presence of a nerve-specific marker, for example by immunofluorescence using a nerve-specific fluorescent marker and confocal microscopy, for nerve growth will indicate the ability of the matrix to support neural in-growth.

Growth supplements can be added to the culture medium, to the matrix or to both in experiments to assess the ability of the matrix to support cell growth. The particular growth supplements employed will be dependent in the type of cells being assessed and can be readily determined by one skilled in the art. Suitable supplements for nerve cells, for example, include laminin, retinyl acetate, retinoic acid and nerve growth factors for nerve cells.

### 3.3 In vivo Testing

In order to assess the biocompatibility of the bio-synthetic matrix and its ability to support cell growth *in vivo,* the matrix can be implanted into an appropriate animal model for immunogenicity, inflammation, release and degradation studies, as well as determination of cell growth. Suitable control animals may be included in the assessment. Examples of suitable controls include, for example, unoperated animals, animals that have received allografts of similar dimensions from a donor animal and/or animals that have received implants of similar dimensions of a standard, accepted implant material.

At various stages post-implantation, biopsies can be taken to assess cell growth over the surface of and/or into the implant and histological examination and immunohistochemistry techniques can be used to determine whether nerve in-growth has occurred and whether inflammatory or immune cells are present at the site of the implant. For example, various cell-specific stains known in the art can be used to assess the types of cells present as well as various cell-specific antibodies, such a anti-neurofilament antibodies that can be used to indicate the presence or absence of nerve cells. In addition, measurement of the nerve action potentials using standard techniques will provide an indication of whether the nerves are functional. *In vivo* confocal microscopic examination can be used to monitor cell and nerve growth in the animal at selected post-operative times. Where appropriate, touch sensitivity can be measured by techniques known in the art, for example, using an esthesiometer. Restoration of touch sensitivity indicates the regeneration of functional nerves.

### 4. APPLICATIONS

The present invention provides a bio-synthetic matrix which is robust, biocompatible and non-cytotoxic and, therefore, suitable for use as a scaffold to allow tissue regeneration *in vivo.* For example, the bio-synthetic matrix can be used for implantation into a patient to replace tissue that has been damaged or removed, for wound coverage, as a tissue sealant or adhesive, as a skin substitute or cornea substitute, or as a corneal veneer. The matrix can be moulded into an appropriate shape prior to implantation, for example it can be pre-formed to fill the space left by damaged or removed tissue. Alternatively, when used as an implant, the matrix may be allowed to form *in situ* by injecting the components into the damaged tissue and allowing the polymers to cross-link and gel at physiological temperature.

In one embodiment of the present invention, the matrix is pre-formed into an appropriate shape for tissue engineering purposes. In another embodiment the matrix is pre-formed as a full thickness artificial cornea or as a partial thickness matrix suitable for a cornea veneer.

The bio-synthetic matrix can be used alone and as such will support the in-growth of new cells *in situ.* Alternatively, the matrix can be seeded with cells prior to implantation and will support the outgrowth of these cells *in vivo* to repair and/or replace the surrounding tissue. It is contemplated that the cells may be derived from the patient, or they may be allogeneic or xenogenic in origin. For example, cells can be harvested from a patient (prior to, or during, surgery to repair the tissue) and processed under sterile conditions to provide a specific cell type such as pluripotent cells, stem cells or precursor cells. These cells can then be seeded into the matrix, as described above and the matrix can be subsequently implanted into the patient.

The matrix can also be used to coat surgical implants to help seal tissues or to help adhere implants to tissue surfaces, for example, through the formation of cross-links between unreacted cross-linking groups on the synthetic polymer component of the hydrogel and primary amino or thiol groups present in the tissue. For example, a layer of the matrix may be used to patch perforations in corneas, or be applied to catheters or breast implants to reduce fibrosis. The matrix may also be applied to vascular grafts or stents to minimise blood or serosal fluid leakage, to artificial patches or meshes to minimise fibrosis and to help adhesion of the implants to tissue surfaces.

The matrix may also be used as a delivery system to deliver a bioactive agent to a particular region in a patient. The bioactive agent can be delivered as a solution together with the synthetic and bio-polymers such that the matrix comprising the bioactive agent can form *in situ,* or the matrix comprising the bioactive agent can be pre-formed and implanted. Once within the body, the bioactive agent may be released from the matrix, for example, through diffusion-controlled processes or, if the bioactive agent is covalently bound to the matrix, by enzymatic cleavage from the matrix and subsequent release by diffusion-controlled processes. Alternatively, the bioactive agent may exert its effects from within the matrix.

In one embodiment of the present invention, the bio-synthetic matrix is used as an artificial cornea. For this application, the matrix is pre-formed as a full thickness artificial cornea or as a partial thickness matrix suitable for a cornea veneer. In accordance with this embodiment, the hydrogel is designed to have a high optical transmission and low light scattering. For example, hydrogels comprising a synthetic p(NiPAAm-co-AAc-co-ASI) terpolymer or p(DMAA-co-ASI) co-polymer cross-linked to collagen have high optical transmission, very low light scattering and are capable of remaining clear up to 55°C.

### 5. KITS

The present invention also contemplates kits comprising the bio-synthetic matrix. The kits may comprise a "ready-made" form of the matrix or they may comprise the individual components required to make the matrix in appropriate proportions (*i.e.* the synthetic polymer and the bio-polymer. The kit may optionally further comprise one or more bioactive agent either pre-attached to the synthetic polymer, or as individual components that can be attached to the synthetic polymer during preparation of the matrix. The kits may further comprise instructions for use, one or more suitable solvents, one or more instruments for assisting with the injection or placement of the final matrix composition within the body of an animal (such as a syringe, pipette, forceps, eye dropper or similar medically approved delivery vehicle), or a combination thereof. Individual components of the kit maybe packaged in separate containers. The kit may further comprise a notice in the form prescribed by a governmental agency regulating the manufacture, use or sale of biological products, which notice reflects approval by the agency of the manufacture, use or sale for human or animal applications.

To gain a better understanding of the invention described herein, the following examples are set forth. It should be understood that these examples are for illustrative purposes only. Therefore, they should not limit the scope of this invention in any way.

### EXAMPLES

### Abbreviations

| | |
|---|---|
| RTT: | rat-tail tendon |
| ddH₂O: | distilled, de-ionised water |
| PBS: | phosphate buffered saline |
| D-PBS: | Dulbecco's phosphate buffered saline |
| AIBN: | azobis-isobutyronitrile |
| NiPAAm: | N-isopropylacrylamide |
| pNiPAAm: | poly(N-iso-propylacrylamide) |
| AAc: | acrylic acid |
| DMAA | N,N-dimethylacrylamide |
| ASI: | N-acryloxysuccinimide |
| pNIPAAm-co-AAc: | copolymer of NiPAAm and AAc |
| poly(NiPAAm-co-AAc-co-ASI): | terpolymer of NIPAAM, AAc and ASI |
| poly(DMAA-co-ASI) | co-polymer of DMAA and ASI |
| GPC: | gel permeation chromatography |
| NMR | nuclear magnetic resonance |
| YIGSR: | amide-terminated pentapeptide (tyrosine-isoleucine-glycine-serine-arginine) |

All gel matrices described in the Examples set out below used sterile collagen I, such as telocollagen (rat-tail tendon, RTT) or atelocollagen (bovine or porcine), which can be prepared in the laboratory or more conveniently is available commercially (for example, from Becton Dickinson at a concentration of 3.0-3.5 mg/ml in 0.02N acetic acid and in 0.012N hydrochloric acid for bovine and porcine collagen). Such collagens can be stored for many months at 4°C. In addition, such collagen solutions may be carefully concentrated to give optically clear, very viscous solutions of 3 - 30 wt/vol % collagen, suitable for preparing more robust matrices.

Collagen solutions are adjusted to physiological conditions, i.e. saline ionic strength and pH 7.2 - 7.4, through the use of aqueous sodium hydroxide in the presence of phosphate buffered saline (PBS). PBS, which is free of amino acids and other nutrients, was used to avoid depletion of cross-linking reactivity by side reactions with -NH₂ containing molecules, so allowing the use of the minimum concentration of cross-linking groups and minimising any risk of cell toxicity. pNiPAAm homopolymer powder is available commercially (for example, from Polyscience). All other polymers were synthesized as outlined below.

### EXAMPLE 1: PREPARATION OF ApNiPAAm-COLLAGEN HYDROGEL

A pNiPAAm-collagen hydrogel was prepared to provide an alternative hydrogel against which the properties of the hydrogels of the present invention could be compared.

A 1 wt/vol% solution of pNiPAAm homopolymer in ddH₂O was sterilised by autoclaving. This solution was mixed with sterile RTT collagen solution [3.0-3.5 mg/ml (w/v) in acetic acid (0.02N in water] (1:1 vol/vol) in a sterile test tube at 4°C by syringe pumping to give complete mixing without bubble formation. Cold mixing avoids any premature gelification or fibrilogenesis of the collagen. The collagen-pNiPAAm was then poured over a plastic dish (untreated culture dish) or a mould (*e.g.* contact lens mould) and left to air-dry under sterile conditions in a laminar flow hood for at least 2-3 days at room temperature. After drying to constant weight (~7 % water residue), the formed matrix was removed from the mould. Removal of the matrix from the mould is facilitated by soaking the mould in a sterile PBS at room temperature. Continued soaking of the free sample in this solution gives a gel at physiological temperature, pH and ionic strength, which was subsequently submitted to testing for cell growth and *in vivo* animal testing (see Examples 6 and 7).

### EXAMPLE 2: PREPARATION OF A SYNTHETIC TERPOLYMER

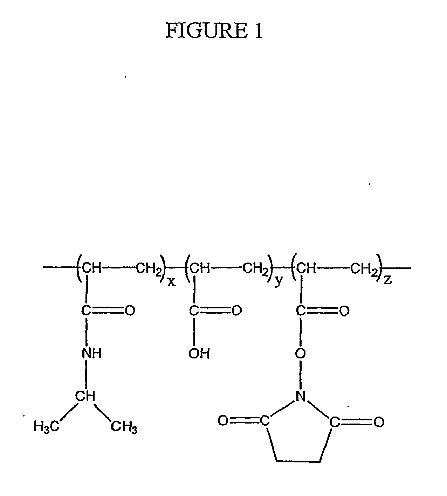

A collagen-reactive terpolymer, poly(NiPAAm-co-AAc-co-ASI) (Figure 1), was synthesised by co-polymerising the three monomers: N-isopropylacrylamide, (NiPAAm, 0.85 mole), acrylic acid (AAc, 0.10 mole) and N-acryloxysuccinimide (ASI, 0.05 mole). The feed molar ratio was 85:10:5 (NiPAAm: AAc: ASI), the free-radical initiator AIBN (0.007 mole/mole of total monomers) and the solvent, dioxane (100 ml), nitrogen purged before adding AIBN. The reaction proceeded for 24 h at 65°C.

After purification by repeated precipitation to remove traces of homopolymer, the composition of the synthesised terpolymer (82% yield) was found to be 84.2:9.8:6.0 (molar ratio) by proton NMR in THF-D₈. The Mₙ and M_{w} of the terpolymer were 5.6 X 10⁴ Da and 9.0 x 10⁴ Da, respectively, by aqueous GPC.

A solution of 2 mg/ml of the terpolymer in D-PBS remained clear even up to 55°C, consistent with a high LCST. A solution of 10 mg/ml in D-PBS became only slightly cloudy at 43°C. Failure to remove homopolymer formed in the batch polymerisation reaction (due to the NiPAAm reactivity coefficient being greater than that of AAc or ASI) from the terpolymer gave aqueous solutions and hydrogels which cloud at ~32°C and above.

### EXAMPLE 3: PREPARATION OF A SYNTHETIC POLYMER COMPRISING A BIOACTIVE AGENT

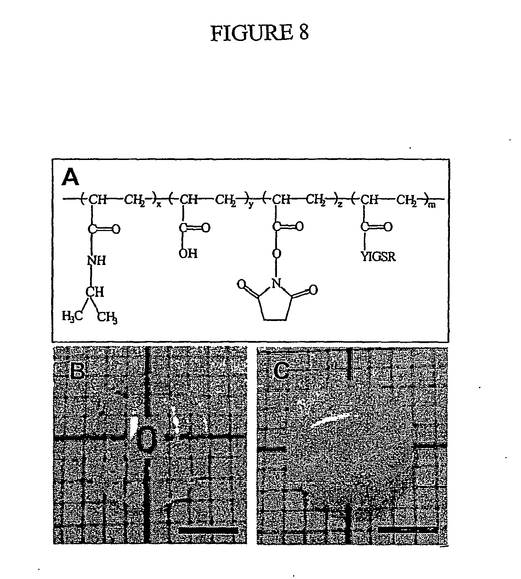

A terpolymer, containing the pentapeptide YIGSR (a nerve cell attachment motif), was synthesised by mixing the terpolymer prepared in Example 2 (1.0 g) with 2.8µg of laminin pentapeptide (YIGSR, from Novabiochem) in N,N-dimethyl formamide. After reaction for 48 h at room temperature (21°C), the polymer product was precipitated out from diethyl ether and then vacuum dried. ASI groups remaining after reaction with the pentapeptide are available for subsequent reaction with collagen. The structure of this polymer is shown in Figure 8A.

### EXAMPLE 4: PREPARATION OFA COLLAGEN-TERPOLYMER HYDROGEL

A cross-linked, terpolymer-collagen hydrogel was made by mixing neutralised 4% bovine atelocollagen (1.2 ml) with the terpolymer prepared in Example 2 [0.34ml (100 mg/ml in D-PBS)] by syringe mixing at 4°C (collagen : terpolymer 1.4 : 1 w/w). After careful syringe pumping to produce a homogeneous, bubble-free solution, aliquots were injected into plastic, contact lens moulds and incubated at room temperature (21°C) for 24 hours to allow reaction of the collagen -NH₂ groups with ASI groups as well as the slower hydrolysis of residual ASI groups to AAc groups. The moulded samples were then incubated at 37°C for 24 hours in 100% humidity environment, to give a fmal hydrogel. The hydrogel contained 95.4 ± 0.1% water, 2.3% collagen and 1.6% terpolymer. Matrices were moulded to have a final thickness between either 150 - 200 µm or 500 - 600 µm. Each resulting hydrogel matrix was removed from its mould under PBS solution and subsequently immersed in PBS containing 1% chloroform and 0.5% glycine. This wash step removed N-hydroxysuccinimide produced in the cross-linking reaction, terminated any unreacted ASI groups in the matrix, by conversion to acrylic acid groups and sterilised the hydrogel matrix. As an alternative, moulded gels may be treated with aqueous glycine to ensure that all ASI are terminated prior to cell contact.

Succinimide residues left in the gels prepared from collagen and terpolymer were below the IR detection limit after washing.

### EXAMPLE 5: PREPARATIONOFAHYDROGEL COMPRISINGA BIOACTIVE AGENT

Cross-linked hydrogels of collagen-terpolymer comprising YIGSR cell adhesion factor were prepared by thoroughly mixing viscous, neutralised 4% bovine collagen (1.2 ml) with terpolymer to which laminin pentapeptide (YIGSR) was covalently attached (from Example 3; 0.34ml, 100 mg/ml) at 4°C, following the procedure described in Example 4.

The YIGSR content of extensively washed gels was 4.3 x 10⁻¹¹ mole/ml (2.6 x 10⁻⁸ g/ml) of hydrated gel quantified by labelling the tyrosine (primary amine-bearing) groups with ¹²⁵I using the Iodogen method and measuring the radioactivity with a standardised gamma counter (Beckman, Gamma 5500). The final, total polymer concentration in each hydrated, PBS-equilibrated hydrogel was 3.4 w/v % (comprising collagen and YIGSR terpolymer at 2.0 and 1.4 w/v %, respectively).

### EXAMPLE 6: COMPARISON OF THE PHYSICAL PROPERTIES OF HYDROGEL MATRICES

Collagen thermogels are frail and readily collapse and break and are obviously opaque (see Figure 8C). Collagen thermogels were prepared by neutralization of collagen and casting in the same moulds as described above in Examples 4 and 5. The moulded collagen was then incubated, first for 24 h at 21°C then at 37°C, to spontaneously form translucent thermogels (produced by self association of collagen triple helices into micro-fibrils).

The permeability coefficient of glucose in PBS (pH 7.4) through hydrogels prepared as described in Examples 5 was calculated from measurements in a permeation cell by periodically removing aliquots of permeate, adding adenosine triphosphate and converting glucose to glucose-6-phosphate with the enzyme hexokinase. The latter was reacted with nicotinamide adenine dinucleotide in the presence of dehydrogenase and the resultant reduced dinucleotide quantified by its UV absorption at 340 nm in solution (Bondar, R. J. & Mead, D. C. (1974) Clin Chem 20, 586-90). Topographies of hydrogel surfaces, fully immersed in PBS solution, were examined by atomic force microscopy (Molecular Image Co., USA) in the "contact" mode. Pore sizes from this technique were compared with average pore diameters calculated from the PBS permeability of the hydrogels as previously described (Bellamkonda, R., Ranieri, J. P. & Aebischer, P. (1995) J Neurosci Res 41, 501-9). The hydrogels had refractive indices (1.343 ± 0.003) comparable to the tear film (1.336-1.357) in the human eye (Patel, S., Marshall, J. & Fitzke, F. W., 3rd (1995) J Refract Surg 11, 100-5). They showed high optical clarity compared to matrices that contain only collagen (Fig. 8B and C). The hydrogels had pore diameters of 140-190 nm (from both atomic force microscopy and PBS permeability) and a glucose diffusion permeability coefficient of 2. 7x10⁻⁶ cm²/s, which is higher than the value for the natural stroma (~0.7x10⁻⁶ cm²/s, calculated from published diffusion (2.4x10⁻⁶ cm²/s) and solubility (0.3) coefficients (McCarey, B. E. & Schmidt, F. H. (1990) Curr Eye Res 9, 1025-39)).

The following properties of the hydrogels prepared as described in Examples 4 and 5 indicate that they are cross-linked:
- water insoluble,
- strong enough to support surgical manipulation with suture thread and needle, and attachment to a human corneal ring
- relatively flexible in handling
- demonstrate an increase in stress at failure and apparent modulus during tensile testing by over x2 on going from ASI/-NH₂ equivalent ratio of 0.5 to 1.5.

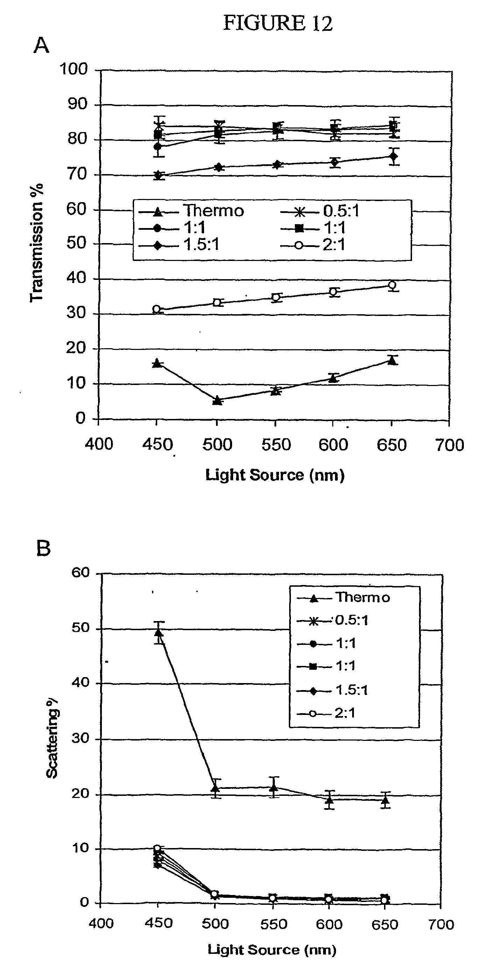

Matrices prepared as described above, but with varying ratios of collagen amine groups to ASI groups in the synthetic polymer had high optical transmission and low scatter in the visible region (Fig. 8B, 12 and 13). In contrast, the collagen thermogel, prepared from collagen as described above, had low transmission and high scatter, consistent with its opaque appearance (Fig. 8C, 12). Such thermogel matrices with up to 3 wt/vol collagen were too weak to allow mounting for suture pull-out testing.

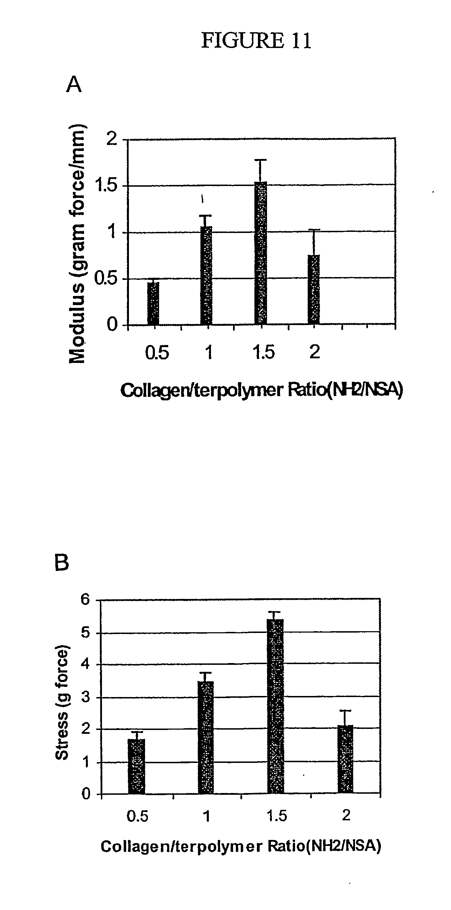

Quantitative characterisation of the hydrogels came from the use of suture pull-out measurements on samples moulded into the shape and thickness of a human cornea. This involved insertion of two diametrically opposed sutures, as required for the first step in ocular implantation, and pulling these two sutures apart at 10 mm/min on an Instron Tensile Tester, a procedure that is well established for the evaluation of heart valve components. The sutures employed were 10-0 nylon sutures. Strength at rupture of the gel is calculated, together with elongation at break and elastic modulus. Modulus and stress at failure from suture pull-out measurments showed that maxima were reached at specific collagen amine to ASI group ratios (Figure 11).

The hydrogels prepared as described in Examples 4 and 5 have high optical transmission and very low light scattering, comparable to the human cornea, as measured with a custom-built instrument that measures transmission and scatter [Priest and Munger, Invest. Ophthalmol. Vis. Sci. 39: S352 - S361 (1998)]. Back scattering and transmission of light across the visible region for hydro gels prepared as in Example 4 showed excellent performance except at high terpolymer concentrations (high collagen amine to terpolymer ASI ratios, Fig. 12). Similarly, a thermogel (free of cross-linking synthetic polymer) had a very low transmission and high back scattering (Fig. 12). The hydro gels described in Example 5 also showed excellent performance in this analysis as shown in Fig. 12 (1:1 ratio of collagen to terpolymer-pentapeptide is represented by the solid squares).

In contrast, collagan-pNiPAAm homopolymer gels (as described in Example 1; 1.0 : 0.7 to 1.0 : 2.0 wt/wt) were opaque at 37°C. In addition, both collagen and pNiPAAm extracted out from this hydrogel into PBS (over 50 wt % loss in 48 h).

### EXAMPLE 7: IN VIVO TESTING OF VARIOUS BIO-SYNTHETIC MATRICES

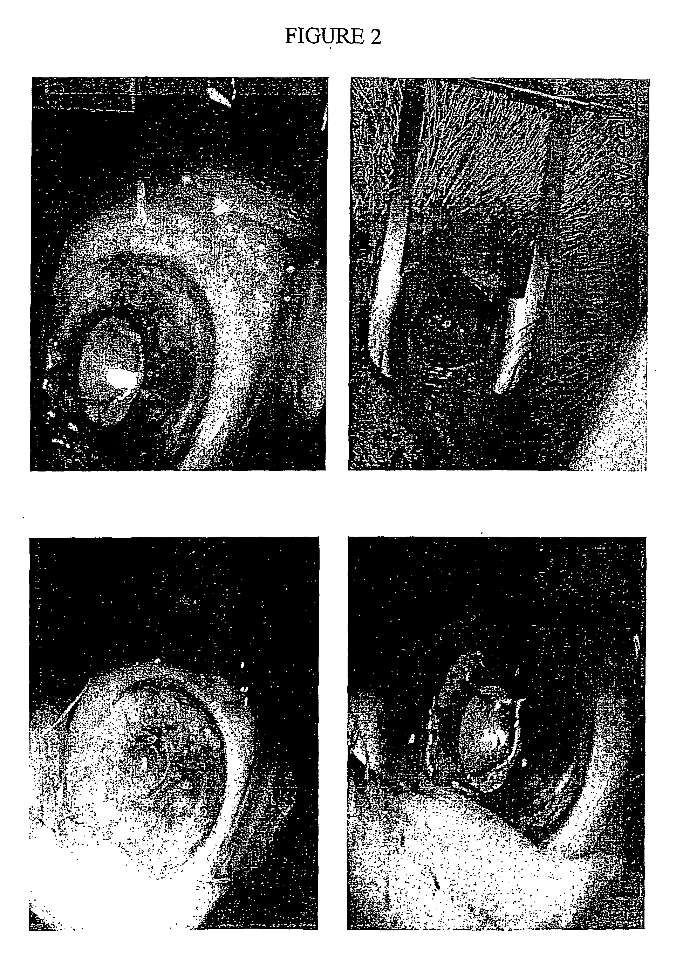

Hydrogels formed as described in Examples 1, 4 and 5 were moulded to form artificial corneas and implanted into the eyes of pigs (Figure 2).

As *in vivo* corneal implants, the gels from Example 1 exude white residue after 5 to 6 days implanted in pigs' eyes.

The hydrogel prepared from 4% collagen and pentapeptide-terpolymer as described in Example 5 demonstrated good biocompatibility as did the collagen-terpolymer hydrogel prepared as described in Example 4. More rapid, complete epithelial cell overgrowth and formation of multiple layers occurred when the former hydrogel was used, as compared to collagen- terpolymer hydrogel which showed slower, less contiguous, epithelial cell growth, without formation of multiple layers.

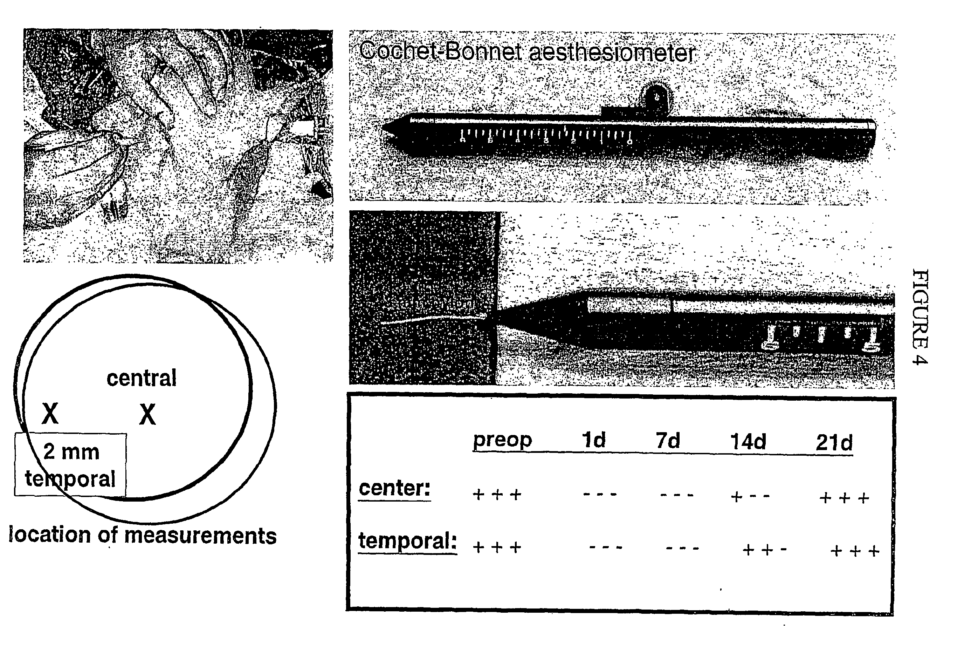

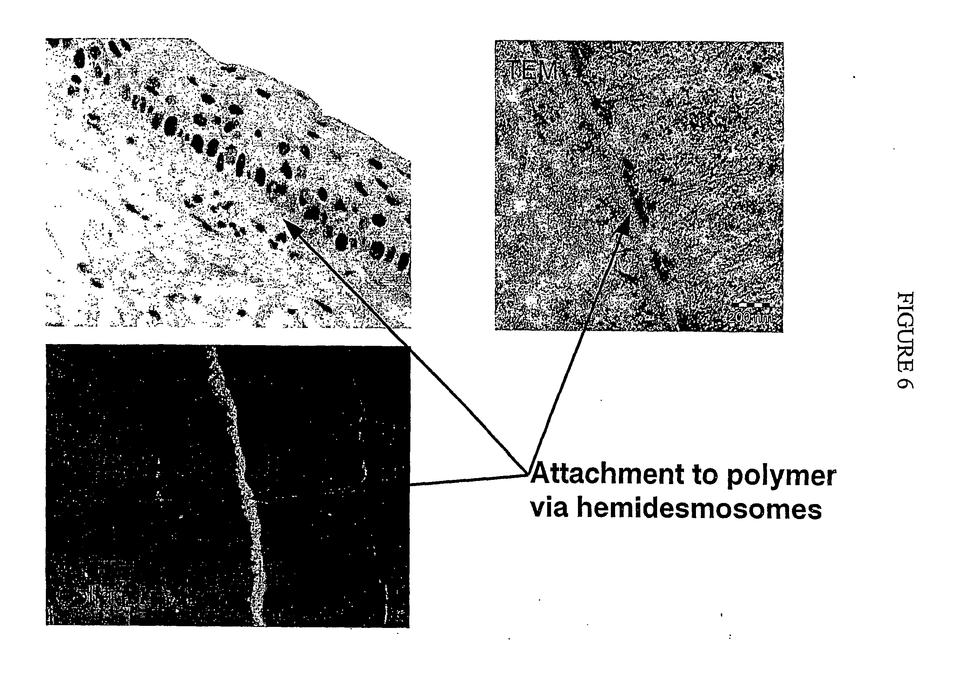

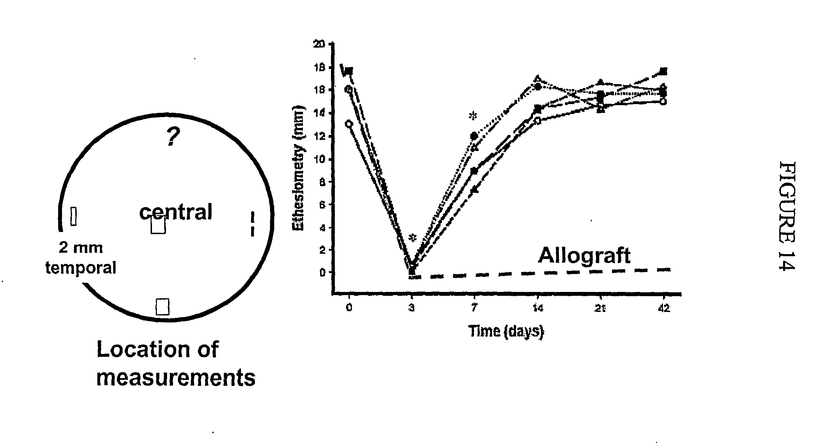

*In vivo,* confocal microscope images of full thickness hydrogel prepared from collagen and the pentapeptide-terpolymer (from Example 5; final concentration: collagen 2.3 wt %; terpolymer + pentapeptide 1.6 wt %) and implanted into a pig's eye showed that epithelium cells grew over this matrix and stratified. A basement membrane was regenerated and hemidesmosomes, indicating a stably anchored epithelium, were present. Stromal cells were found to spread inside the matrix after only three weeks. The implants became touch sensitive within 3 weeks of implantation (Cochet-Bonnet Aesthesiometer) indicating functional nerve in-growth (Figures 4 and 14). Nerve in-growth was also observed directly by confocal microscopy and histology. No clinical signs of adverse inflammation or immune reaction were observed over an 8 week period following implantation. See Figures 2, 3 and 5-7.

### In more detail:

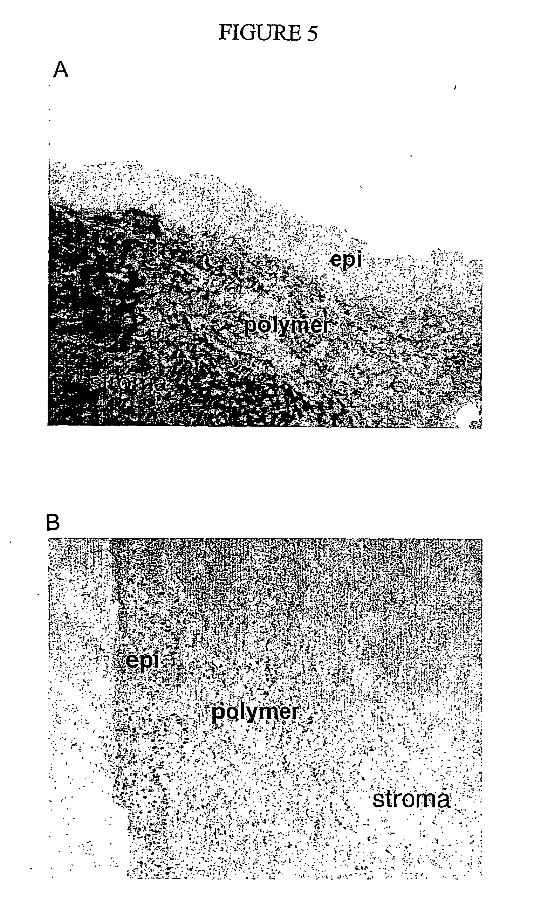

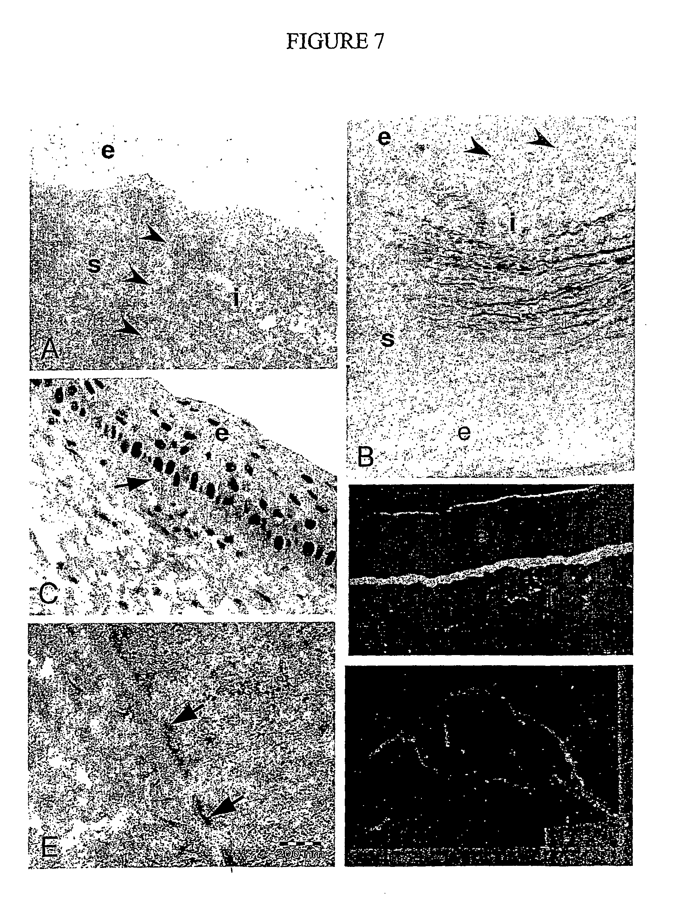

Figure 5 shows morphological and biochemical assessment of a section through the pig cornea at 3 weeks post-implantation (A) picro-sirius stain for collagen and (B) H&E stain for cells. Figure 7 shows (A) a section through the pig cornea at 3 weeks post-implantation, stained with picro-sirius red, which demonstrates the stromal-implant interface (arrowheads). The implant surface has been re-covered by a stratified epithelium. (B) a similar section at 8 weeks post-implantation. Stromal cells have moved into the implant and the implant appears to have been replaced by tissue sub-epithelially (arrows). (C) a higher magnification of the epithelium (H & E stained) showing the regenerated basement membrane (arrow). (D) a corresponding section stained with anti-type VII collagen antibody that recognizes hemidesmosomes attached to the basement membrane (arrow). (E) the hemidesmosomes (arrows) attached to the underlying basement membranes are clearly visualized by transmission electron microscopy (TEM). (F) a flat mount of the pig cornea showing nerves (arrowheads) within the implant, stained with an anti-neurofilament antibody.

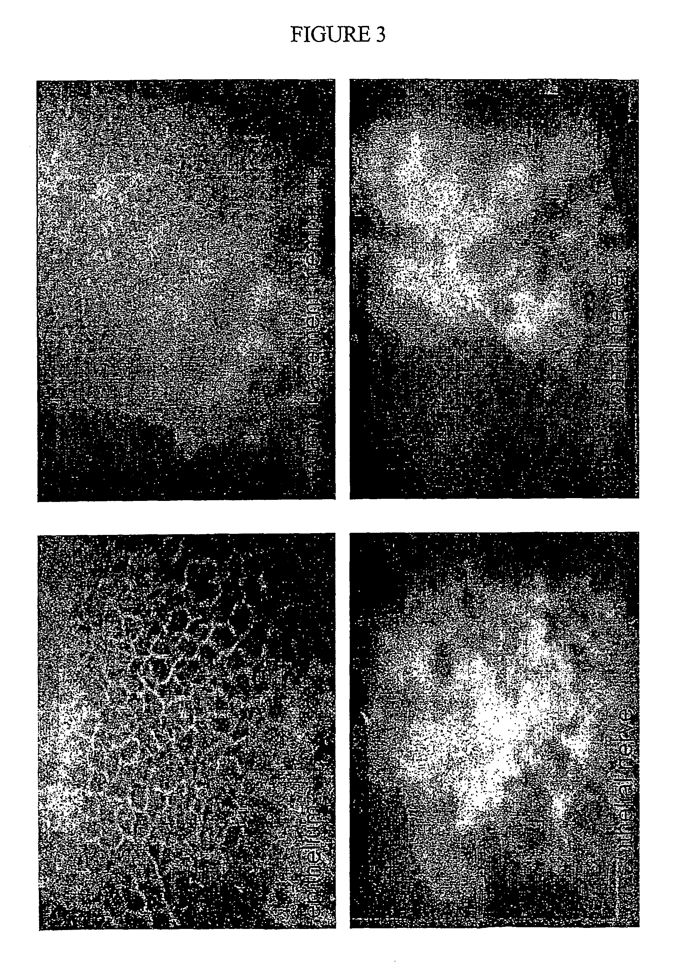

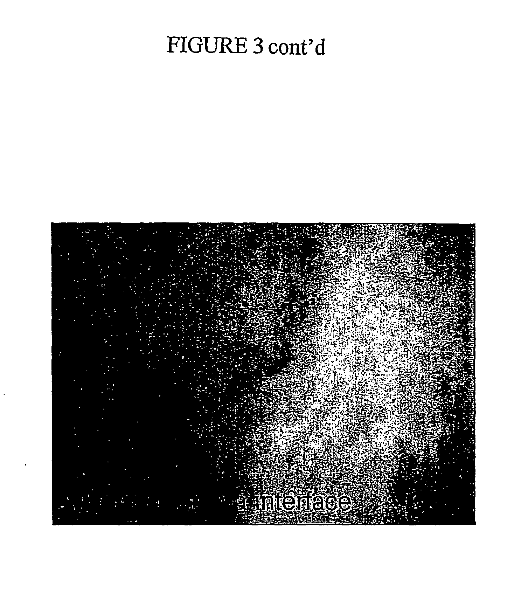

Figure 3 shows whole mount confocal microscopic images of pigs corneas at 6 weeks post-operation showing a regenerated corneal epithelium and basement membrane on the surface of the implant. *In vitro* nerve growth patterns within the collagen-terpolymer composite and within the underlying host stroma are shown, as are ingrowing stromal cells.

Restoration of touch sensitivity was rapid (< 14 days post-operative) in comparison with minimal restoration in the transplanted allograft over the same time period for an additional six animals that received allografts of donor pig corneas of similar dimensions (Figure 14).

### EXAMPLE 8: DEPOSITION OF COLLAGEN-TERPOLYMER MATRICES INTO RODENT BRAIN

Following euthanasia, the whole brain of each mouse or rat used was excised and placed within a sterotaxic frame. Either two microlitres (2 ml) or three microlitres (3 ml) of hydrogel containing collagen, terpolymer-pentapeptide at either 0.33% collagen - 0.23% terpolymer or 0.63% collagen-0.44% terpolymer was injected over a period of 6 to 10 min, respectively, into each individual mouse brain, at the following coordinates: 0.3 mm from bregma, 3.0 mm deep and 2.0 mm from the midline. For rats, four to six microlitres of hydrogel was injected over 10 min. into each brain, at 0.7-0.8 mm from bregma, 6 mm deep and 4 mm from the midline. The hydrogel samples were mixed with Coomassie blue dye for visualization.

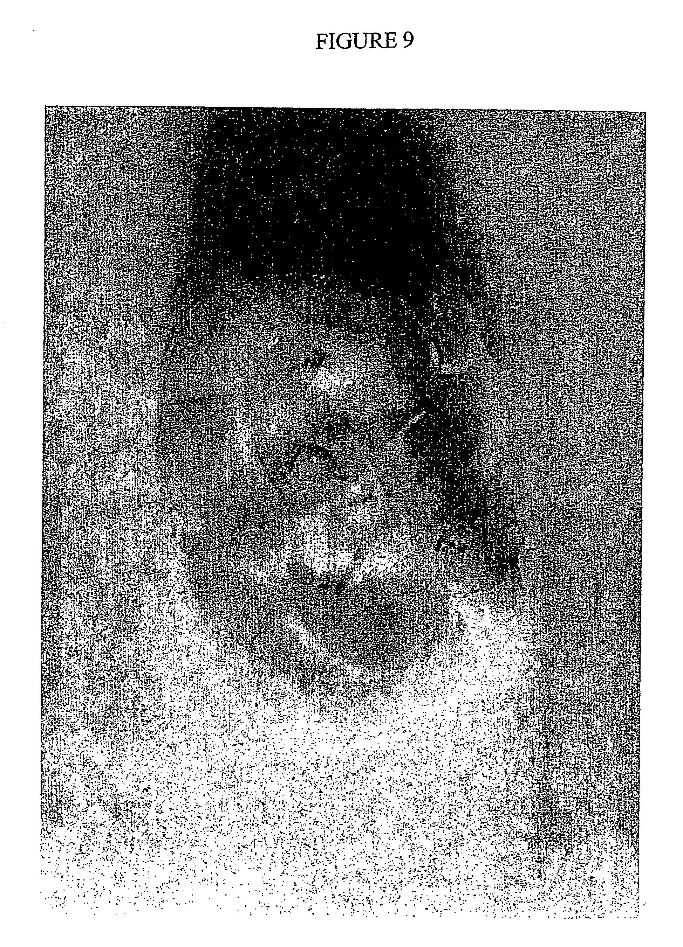

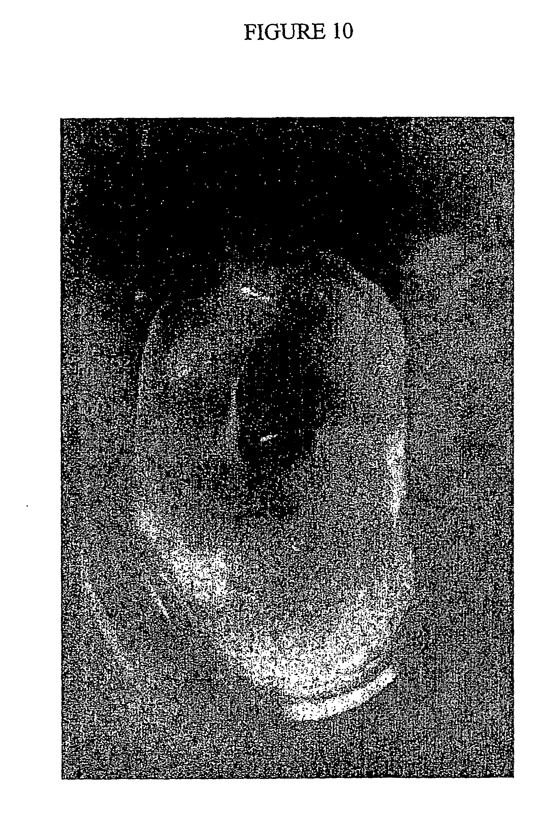

Results indicate successful direct, precise delivery of small amount of the hydrogel into the stratum of the brain, in these samples (Figures 9 and 10). This suggests that it is possible to use the hydrogel as a delivery system for cells or drugs into specific locations at very small volumes.

### EXAMPLE 9: IN VIVO TESTING OFA HYDROGEL COMPRISING A BIOACTIVE AGENT

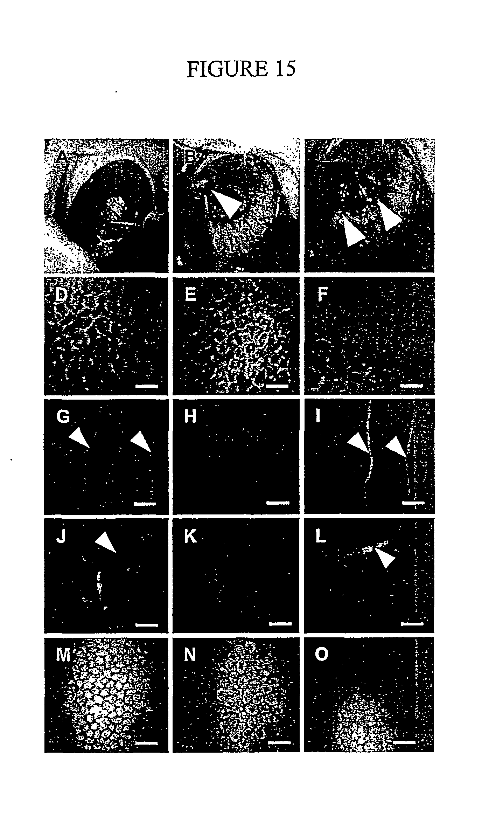

Sterile hydrogels prepared as described in Example 5 were thoroughly rinsed in PBS before implantation. Following the Association for Research in Vision and Ophthalmology guidelines for animal use, each tissue engineered (TE) corneal matrix (5.5 mm in diameter and 200 ± 50 µm thick) was implanted into the right cornea of a Yucatan micro-pig (Charles River Wiga, Sulzbach, Germany) (see Fig. 15A-C). Contralateral unoperated corneas served as controls. Under general anaesthesia, a partial-thickness 5.0 mm diameter circular incision was made using a Barraquer trephine (Geuder, Heidelberg, Germany). Host corneal tissue was removed and replaced with an implant 0.50 mm larger in diameter to allow adequate wound apposition between the graft and host tissue. After surgery, an amniotic membrane was sutured over the entire corneal surface for one week to keep implants in place. In sutured samples, implants were sutured into the host tissue using 8 interrupted 10-0 nylon sutures. Post-operative medication consisted of dexamethasone (qid) and gentamycin (qid) for 21 days. n= 3 pigs with sutures and 3 without sutures.

Follow-ups were performed daily on each pig up to 7 days post-operative, and then weekly. Examinations included slit-lamp examination to ensure corneas were optically clear, sodium fluorescein staining to assess epithelial integrity and barrier function (Josephson, J. E. & Caffery, B. E. (1988) Invest Ophthalmol Vis Sci 29, 1096-9), measurements of intraocular pressure to ensure that corneas were not blocking aqueous humour flow, and *in vivo* confocal microscopic examination (ConfoScan3, Nidek, Erlangen, Germany) to assess cell and nerve in-growth. Corneal touch sensitivity was measured using a Cochet-Bonnet esthesiometer (Handaya Co., Tokyo, Japan) at five points within the implant area of each cornea (four peripheral, one central) as previously described (Millodot, M. (1984) *Ophthalmic Physiol Opt* **4**, 305-18). Animals that received allografts of pig donor corneas were also similarly evaluated.

For immunohistochemistry and histopathological examination, tissues and constructs were fixed in 4% PFA in 0.1M PBS. For nerve immunolocalization, flat mounts were permeabilized with a detergent cocktail (Brugge, J. S. & Erikson, R. L. (1977) Nature 269, 346-8) (150 mM NaCl, 1 mM ethylenediamine tetraacetic acid, 50 mM Tris, 1% Nonidet P-40, 0.5% sodium deoxycholate, 0.1% sodium dodecyl sulphate), blocked for non-specific staining with 4% foetal calf serum in PBS and incubated in anti-neurofilament 200 antibody (Sigma, Oakville, Canada). They were then incubated with FITC or Cy3-conjugated secondary antibodies (Sigma; Amersham, Baie D'Urfé, Canada, respectively) and visualization by confocal microscopy.

For histology and further immunohistochemistry, samples were processed, paraffin embedded and sectioned. Sections were stained with haematoxylin and eosin (H&E) for histopathological examination (Jumblatt, M. M. & Neufeld, A. H. (1983) Invest Ophthalmol Vis Sci 24,1139-43). Immunofluorescence was performed as described above on deparaffinized sections for expression of type VII collagen (Sigma, Munich, Germany), a hemidesmosome marker (Gipson, I. K., Spurr-Michaud, S. J. & Tisdale, A. S. (1988) Dev Biol 126, 253-62). Immunohistochemical staining using peroxidase-diaminobenzidine (DAB) visualization was performed with the following: with AE1/AE3 antibody (Chemicon, Temecula, CA, USA) for epithelial markers, anti-vimentin antibody (Roche, Laval, Canada) for stromal fibroblasts, anti-smooth muscle actin antibody, 1A4 (Cell Marque, Austin, TX) for activated stromal fibroblasts (myofibroblasts) and SP1-D8 antibody (DHSB, Iowa, USA) for procollagen 1 synthesis (to localize sites of *de novo* collagen synthesis). CD15 and CD45 staining for immune cells (Becton-Dickinson, Oakville, Canada) was performed using the ARK peroxidase kit (DAKO, Mississauga, Canada) to pre-conjugate the primary antibodies to their respective secondary antibodies and peroxidase for visualization. For anti-vimentin, anti-smooth muscle actin and SP8-D1 antibodies, antigen retrieval was preformed by pre-treating with Proteinase-K (2 mg/ml) for 30 min at 37°C prior to incubation in primary antibody. *Ulex europaeus aggultinin* (UEA) lectin staining was used to visualize tear film mucin deposition (Shatos, M. A., Rios, J. D., Tepavcevic, V., Kano, H., Hodges, R. & Dartt, D. A. (2001) Invest Ophthalmol Vis Sci 42, 1455-64). Samples were incubated with biotinylated UEA (Sigma), then reacted with avidin-horseradish peroxidase and visualized with DAB. For transmission electron microscopy (TEM), all samples were treated in conventional fixative, stain and potting resin (Karnovsky's, OsO₄, uranyl acetate, epoxy).

No adverse inflammatory or immune reaction was observed by clinical examination after implantation of either bio-synthetic matrices or pig corneas. Epithelial cell in-growth over the implant was complete by 4 days post-operative. By one week, the regenerated epithelium showed exclusion of sodium fluorescein dye, indicating that the epithelium was intact and had re-established barrier properties. Intraocular pressures were between 10 and 14 mm mercury (Hg) pre-operatively, and 10-16 mm Hg post-operatively throughout the study period of up to 6 weeks, showing that the implants did not block flow of aqueous humour within the eye. Implants remained optically clear (slit-lamp biomicroscopy) and epithelial re-stratification was observed in all animals at 3 weeks post-surgery. Clinical *in vivo* confocal microscopy of the implanted stromal matrices at 3 weeks post-surgery showed a regenerated epithelium (Fig. 15D), newly in-grown nerves (Fig. 15G), and stromal (Fig. 15J) and endothelial cells (Fig. 15M) with cellular morphology mimicking that of un-operated controls (Fig. 15F,I,L,O). Epithelial and endothelial cell morphology in the allografts (Fig. 15E,N) was similar to that of controls. Sub-epithelial and stromal nerves were not observed in the allografts at 3 weeks post-surgery (Fig. 15H,K).

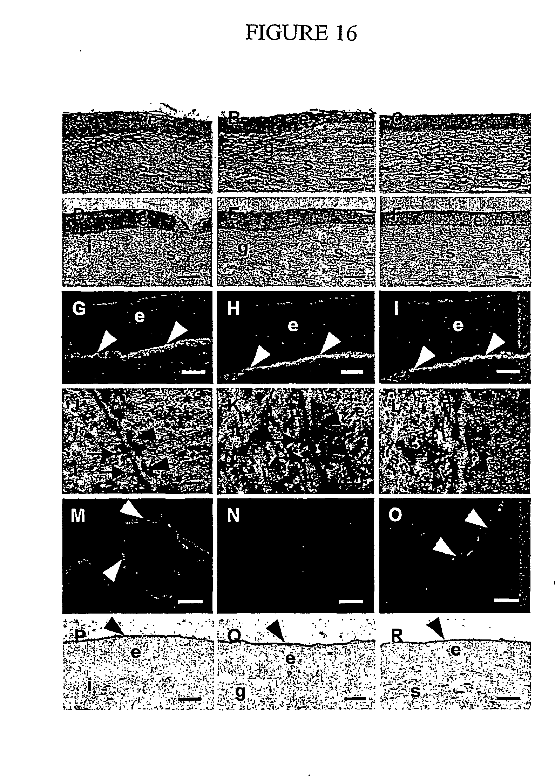

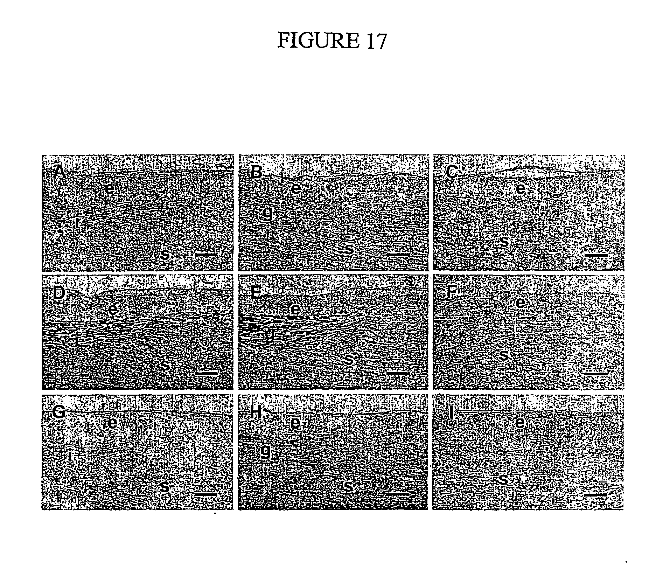

In more detail, Figure 15 shows:
(A-C) lamellar keratoplasty (LKP) procedure on a Yucatan micro-pig. (A): A trephine is used to cut a circular incision of pre-set depth (250 µm) into the cornea. The existing corneal layers are removed and (B) are replaced with a bio-synthetic matrix implant (arrow, 250 µm in thickness), which is sutured in place (C). Sutures are indicated by arrowheads.
(D-O) *In vivo* confocal microscopy of implanted bio-synthetic matrix. (D): confocal image showing regenerated corneal epithelium on the surface of the implant. The corresponding allograft control (E) contains donor epithelium, while the un-operated control (F) has an intact epithelium. (G): Regenerated nerves (arrowheads) are present at the interface between implant and overlying regenerated epithelium. These correspond to the sub-epithelial nerves in the un-operated control (I). In the allograft (H), however, sub-epithelial nerves are absent. (J-L): Stromal cells and branching nerve bundle (arrowhead) deeper within the underlying stroma of corneas with implant (J), allograft (K) and in a corresponding region in the control (L). (M-O): The endothelium in corneas with implant (M), allograft (N) and un-operated controls (O) are intact and show similar morphology.
   Histological sections through corneas with implants showed a distinct but smooth, implant-host tissue interface (Fig. 16A) that resembled that of control corneas that received allografts (Fig. 16B). In both corneas with implants or allografts, the regenerated epithelium was stratified. Detailed examination showed a fully differentiated epithelium that was positively stained by AE1/AE3 antibody markers (Fig. 16D,E), overlying a regenerated basement membrane that was positive for Type VII collagen, a marker for hemidesmosomes at the basement membrane-epithelium interface (Fig. 16G,H). TEM observations indicated morphology consistent with the presence of hemidesmosomes (Fig. 16J,K). In the implants, neurofilament-positive ingrowing nerves had begun to re-establish a sub-epithelial network and showed extension into the epithelial cells (Fig. 16M). However, no sub-epithelial nerves were located in the allografted corneas (Fig. 16N). The tear film was restored in corneas with implants (Fig. 16P) as in the allograft (Fig. 16Q).
   In more detail, Figure 16 shows: post-surgical corneal regeneration.
(A-F) H&E stained sections are shown. Stromal cells are present in the implant (A) and the allograft control (B), and both appear to be seamlessly integrated into the host. (symbols are as follows: e, epithelium; i, implant; g, allograft; s, stroma). (C): Unoperated control. The regenerated epithelium of the implant (D) and donor epithelium of the allograft control (E) expressed cytokeratin differentiation markers, similar to the un-operated control (F).
(G-I): Immunolocalization of type VII collagen, a marker for hemidesmosomes, at the epithelium-implant interface (arrows) in the implant (G), allograft (H) and control (I).
(J-L): TEM of epithelium-implant interface. Hemidesmosome plaques (arrowheads) and anchoring fibrils (arrows) have formed within the bio-synthetic matrix between the epithelial cells and underlying implant (J), emulating the structures normally found at the epithelial-stromal interface as demonstrated in the allograft (K) and control (L).
   Flat mount of cornea showing nerve fibres (arrows) within the implant (M), and un-operated control (O) but absent in the allograft (N), stained with an anti-neurofilament antibody. UEA binding (arrowheads) to the epithelial surface on the implant (P), and allograft (Q) indicate restoration of the tear film in all cases. Un-operated control (R).
   Immunohistochemistry results indicated that cells within both implant and allograft were synthesizing procollagen I. However, more procollagen synthesis occurred in the allografts as indicated by the more intense staining in allografts compared to implants (Fig. 17G,H). Both allografts and implants had stromal cells that were vimentin positive (Fig. 17A,B), indicating a fibroblastic phenotype. Both also showed smooth muscle actin staining and therefore the presence of activated stromal fibroblasts, although the implants showed fewer positive cells than the allografts (Fig. 17D,E).
   In more detail, Figure 17 shows: implant-host integration at 6 weeks post surgery.
(A-C): Staining for procollagen type I. Positive staining is observed in matrix of both the implanted biosynthetic matrix (A) and the allograft control (B) indicating sites of new collagen deposition. Unoperated control (C) has no new collagen synthesis.
(D-F): Staining for vimentin throughout stroma identifies stromal fibroblasts.
   Staining throughout the implanted biosynthetic matrix (D) demonstrates cell invasion. Cells may also been seen within the implanted allograft (E) and throughout the un-operated control (F).
(G-I): Smooth muscle actin staining indicates activated myofibroblasts and the potential for scarring. In the biosynthetic matrix implant (G), staining is occasionally present in the biosynthetic matrix, but is not found in the host stroma, nor in the transition zone between host and implant. Positive staining in the allograft implanted cornea (H) is identified both in the allograft, and the transition zone, but not in the intact host stroma. (I): Un-operated control.

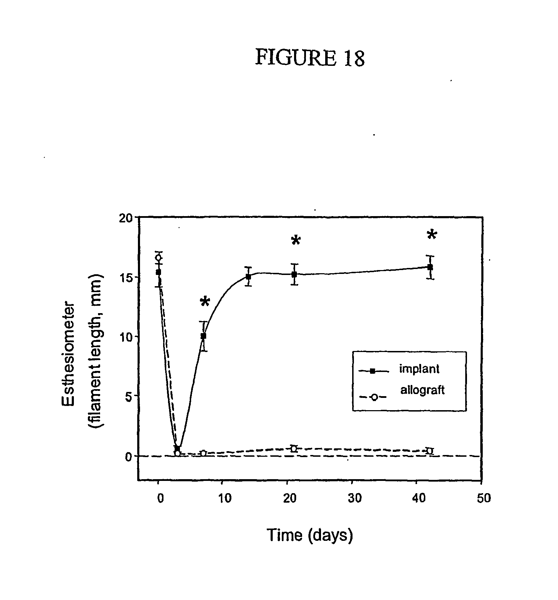

Comeal touch sensitivity measured at 5 points on the corneal implant in 3 pigs pre- and post-operatively using a Cochet-Bonnet esthesiometer, showed a dramatic drop in touch sensitivity after surgery. However, recovery occurred between 7 and 14 days and by 21 days post-operative, sensitivity had returned to pre-operative levels (Fig. 18; All groups, n = 3. **P* < 0.01 by repeated measures ANOVA with Tukey 2-way comparisons). Touch sensitivity returned at the same rate and to the same plateau level at all peripheral and central points tested on the implant. In control animals that had received donor corneal allografts, however, the corneas remained anaesthetic over the six-week period (Fig. 18).

Implants recovered after 6 weeks in vivo were examined by infrared spectroscopy (Midac M, FTIR spectrometer, ZnSe beam condenser and diamond cell) and clearly indicated the presence of the terpolymer.

### EXAMPLE 10: PREPARATION OF A SYNTHETIC CO-POLYMER

A poly(DMAA-*co*-ASI) co-polymer was synthesised by co-polymerization of the monomers: N,N-dimethyl acrylamide, (DMAA) and N-acryloxysuccinimide (ASI). The feed molar ratio was 95:5 (DMAA: ASI). The free-radical initiator AIBN and the solvent, dioxane, were nitrogen purged prior to use and polymerisation reaction proceeded at 70°C for 24h.

After purification by repeated precipitation to remove traces of homopolymer, the composition of the synthesized copolymer (70% yield) was found to be 94.8:5.2 (molar ratio) by proton NMR. Molecular mass (Mₙ) was determined at 4.3 x 10⁴, by aqueous GPC. Polydispersity (PD; M_{w}/Mₙ) =1.70 was also determined by GPC.

### EXAMPLE 11: PREPARATION OF A COLLAGEN-CO-POLYMER HYDROGEL

A cross-linked collagen-co-polymer hydrogel was prepared by mixing neutralized 5% bovine collagen (1.0 ml) with the synthetic co-polymer prepared in Example 9 [0.2ml (200 mg/ml in D-PBS)] by syringe mixing. After careful syringe pumping to produce a homogeneous, bubble-free solution, aliquots were injected into plastic, contact lens moulds and incubated at room temperature for 24 hours to allow reaction of the collagen -NH₂ groups with ASI groups in the co-polymer as well as the slower hydrolysis of residual ASI groups to AAc groups.

The moulded samples were then incubated at 37°C for 24 hours in a 100% humidity environment to provide the final hydrogel. At gelation, the hydrogel contained 94.8% water, 2.9% collagen and 2.3% synthetic co-polymer. Matrices were moulded to have a final thickness between either 150 - 200 µm or 500 - 600 µm. Each resulting hydrogel matrix was removed from its mould under PBS solution and subsequently immersed in PBS containing 1% chloroform and 0.5% glycine. This wash step removed N-hydroxysuccinimide produced in the cross-linking reaction and terminated any residual ASI groups in the matrix, by conversion to acrylic acid groups.

Succinimide residues left in the gels prepared from collagen and copolymer were below the IR detection limit after washing.

### EXAMPLE 12:PHYSICAL PROPERTIES OF COLLAGEN-CO-POLYMER HYDROGEL

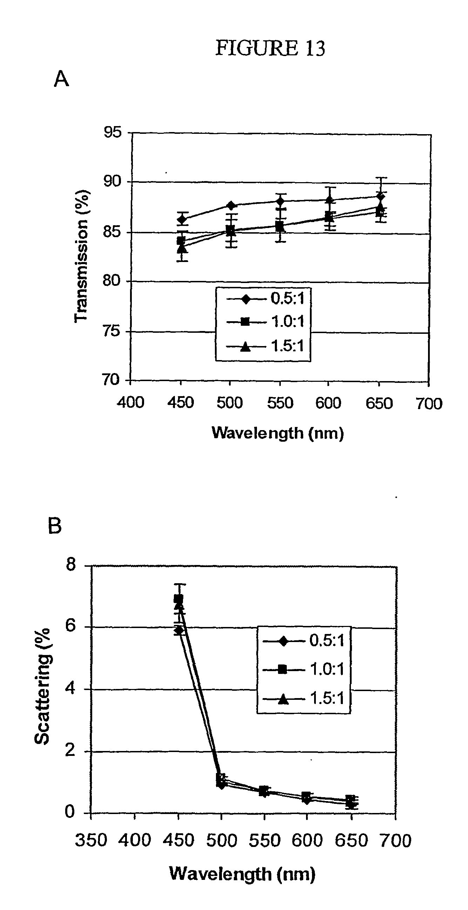

Light back scattering and light transmission across the visible region and with white light for hydrogels prepared as in Example 10 as a function of collagen amine to copolymer ASI ratios is shown in Fig 13A and B.

The copolymer from Example 10 and its hydrogels had no detectable cloud point (LCST) at up to 60°C.

### EXAMPLE 13: BIOLOGICAL PROPERTIES OF VARIOUS HYDROGELS

### 11.1 Biocompatibility

Three 12 mm diameter and 650 µm thick discs each of collagen-poly(DMAA-co-ASI), collagen-poly(NiPAAm-co-AAc-co-ASI)-pentapeptide and 3% collagen hydrogels were soaked for 30 minutes in PBS. They were each laid onto a 12 mm membrane insert commercially available for a culture dish and adhered to the membrane with a thin coating of gelatin. After drying for 10 minutes, 1 X 10⁴ human corneal epithelial cells (HCEC) cells suspended a serum-free medium containing epidermal growth factor (Keratinocyte Serum-Free Medium (KSFM; Life Technologies, Burlington, Canada)) were added to the top of the gels, and KSFM without cells was added to the underlying well. Cultures were incubated at 37°C with 5% CO₂.

Within 12 hours the cells had adhered to the surface of the matrix in all samples. Medium was changed every second day with KSFM added to the inserts and to the outside wells. HCEC were grown to confluence on the gels and reached confluence on the same day (5 days). The medium in the inserts and surrounding wells was replaced by a serum-containing medium (modified SHEM medium (Jumblatt, M. M. & Neufeld, A. H. (1983) Invest Ophthalmol Vis Sci 24, 1139-43)). After 2 more days, the medium was removed from the inserts, and the volume of SHEM in the underlying wells reduced to 0.5 ml. The epithelium was allowed to stratify for a further 7 days and the layer of cells visualized.

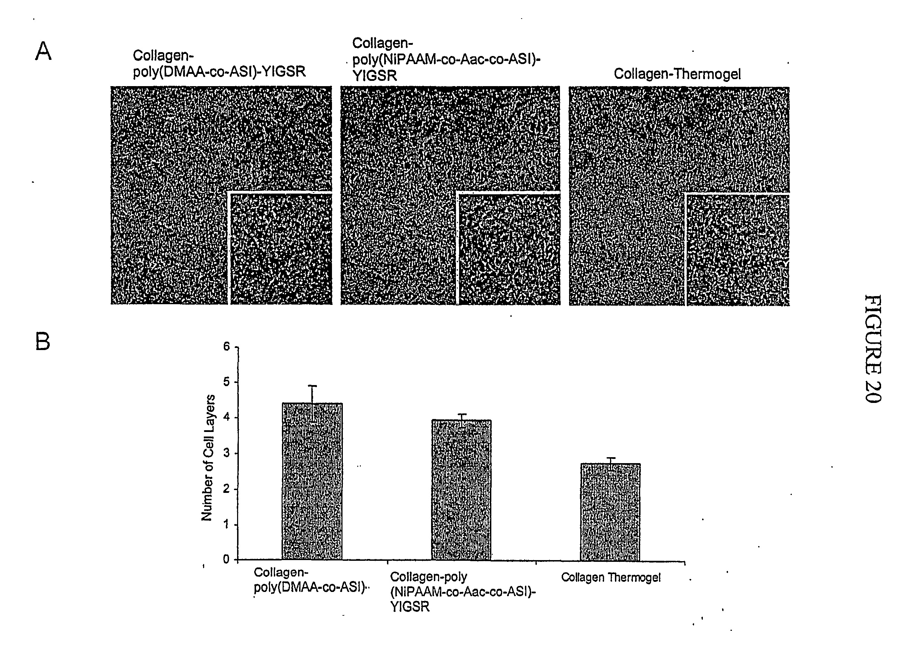

After 7 days, the membranes were fixed in 4% paraformaldehyde in PBS for 30 minutes at 4°C. Samples were prepared for cryosectioning by equilibration in 30% sucrose in PBS followed by flash freezing in a 1:1 mixture of 30% sucrose in PBS and OCT. These were cryosectioned to 13 µm and the structure visualized by HandE staining. The number of cell layers in the stratified epithelium was determined by counting nuclei and identifying cell borders. The collagen thermogel attained an epithelial thickness of approximately 2 cells, which contrasts poorly with the human corneal epithelium that contains between about 5 and 7 cell layers. HCEC cultured and induced to stratify on collagen-p(DMAA-co-ASI) and collagen-p(NiPAAm-co-AAc-co-ASI)-pentapeptide, however, resulted in an epithelium about 4.5 cell layers thick that included apparently keratinized outer layers suggesting appropriate differentiation of the epithelium (Fig. 20).

### 11.2. Innervation of Hydrogel

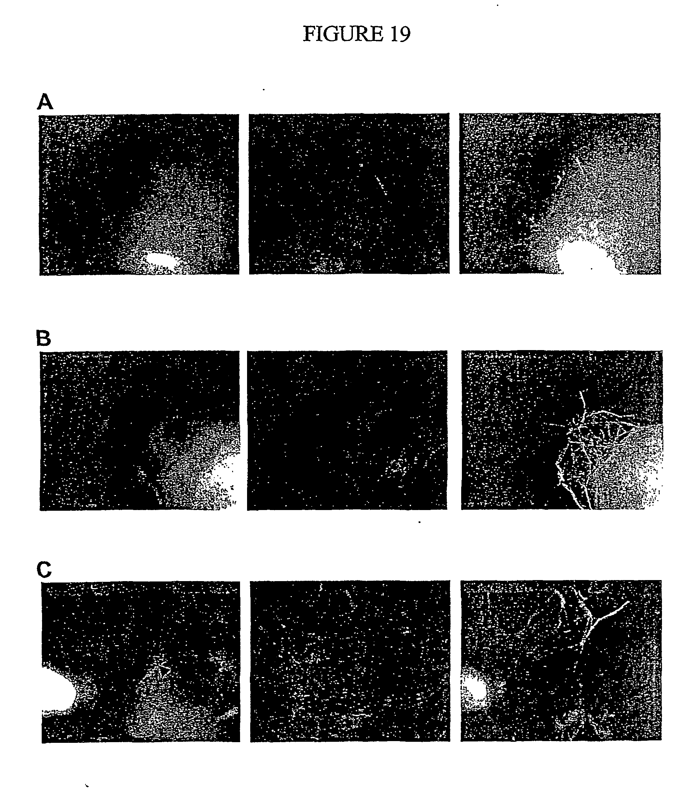

Twelve millimeter diameter and 650 µm thick discs each of collagen-p(DMAA-co-ASI), collagen-p(NiPAAm-co-AAc-co-ASI)-pentapeptide and 3% collagen thermogel were soaked for 30 minutes in PBS. Discs were laid in a 6 cm culture dish, and four 1 mm holes bored through each. The holes were filled a third of the way up with a plug of 0.3% collagen cross-linked with glutaraldehyde and quenched with glycine. After 10 minutes, dorsal root ganglions from E8 chicks were dipped in the same collagen mixture and placed in the holes. The holes were filled the rest of the way with cross-linked collagen, and allowed to set for 30 minutes at 37°C. Cultures were grown for 4 days in KSFM supplemented with B27, N2, and 1 nM retinoic acid for 4 days and neurite extension monitored by brightfield microscopy. The innervated discs were fixed in 4% paraformaldehyde in PBS for 30 minutes room temperature, stained for NF200 immunoreactivity, and visualized by immunofluorescence. Localization was visualized on the surface and in the centre of the polymer disc. While there was some neurite extension over the surface of the collagen thermogel, none could be seen extending into the thermogel itself. In the hydrogels, neurites could be seen extending into the matrix. As well, in both the hydrogels extensive innervation could be seen over the surface of the matrix suggesting a better surface innervation than occurred with the collagen thermogel (Fig. 19; A depicts the collagen thermogel, B depicts the collagen-p(NiPAAm-co-AAc-co-ASI)-pentapeptide hydrogel and C depicts the collagen-p(DMAA-co-ASI) hydrogel). The left column represents immunofluorescent visualizations of the middle of the polymers stained for the nerve neurofilament marker - NF200. The middle column depicts a brightfield view of the surface of the polymer with the neurites extending from the ganglion source. The right column represents an immunofluorescent visualization of the same surface view of the polymer stained for NF200 immuno-reactivity. The arrows indicate neurites extending in the middle of the polymer. The intact human cornea demonstrates both sub-epithelial surface and deep nerves suggesting that these matrices are both biocompatible to nerves and can emulate the corneal stroma.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### EMBODIMENTS OF THE INVENTION

1. A synthetic co-polymer comprising one or more N-alkyl or N,N-dialkyl substituted acrylamide co-monomer, one or more hydrophilic co-monomer and one or more acryl- or methacryl- carboxylic acid co-monomer derivatised to contain a pendant cross-linkable moiety, said synthetic polymer having a number average molecular mass between about 2,000 and about 1,000,000.
2. The synthetic co-polymer according to aspect 1, wherein:
   (a) said N-alkyl or N,N-dialkyl substituted acrylamide co-monomer has a structure of Formula I: 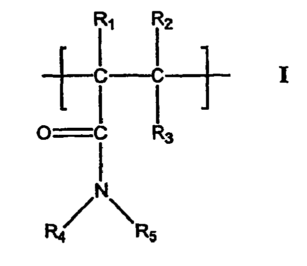 wherein:
      R₁, R₂, R₃, R₄ and R₅ are independently selected from the group of: H and lower alkyl;
   (b) said hydrophilic co-monomer has a structure of Formula II: 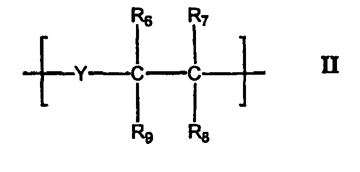 wherein:
      Y is O or is absent;
      R₆, and R₇ are independently selected from the group of: H and lower alkyl;
      R₈ is H, lower alkyl or -OR', where R' is H or lower alkyl; and
      R₉ is H, lower alkyl or -C(O)R₁₀, and
      R₁₀ is -NR₄R₅ or-OR", where R" is H or CH₂CH₂OH; and
   (c) said acryl- or methacryl- carboxylic acid co-monomer has a structure of Formula III: 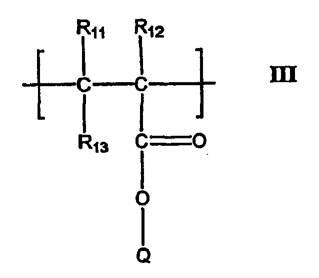
   wherein:
   R₁₁, R₁₂ and R₁₃ are independently selected from the group of: H and lower alkyl, and
   Q is N-succinimido, 3-sulpho-succinimdo (sodium salt), N-benzotriazolyl, N-imidazolyl and *p*-nitrophenyl.
3. The synthetic co-polymer according to aspect1 or 2, wherein said one or more N-alkyl or N,N-dialkyl substituted acrylamide co-monomer and said one or more hydrophilic co-monomer are the same.
4. The synthetic co-polymer according to aspect1 or 2, wherein said one or more N-alkyl or N,N-dialkyl substituted acrylamide co-monomer and said one or more hydrophilic co-monomer are different.
5. The synthetic co-polymer according to any one of aspects i to 4, wherein said alkyl or lower alkyl is a straight or branched chain alkyl group having between one and eight carbon atoms.
6. The synthetic co-polymer according to any one of aspects 1 to 4, wherein said alkyl or lower alkyl is cycloalkyl group having between three and six carbon atoms.
7. The synthetic co-polymer according to aspect 2, wherein the combined molar ratio of N-alkyl or N,N-dialkyl substituted acrylamide co-monomer and hydrophilic co-monomer is between about 50% and about 99.5% and the molar ratio of derivatised acryl- or methacryl- carboxylic acid co-monomer is between about 0.5% and about 50%, wherein the sum of said molar ratios is 100%.
8. The synthetic co-polymer according to aspect 3, wherein the molar ratio of N-alkyl or N,N-dialkyl substituted acrylamide co-monomer is between about 50% and about 90%, the molar ratio of the hydrophilic co-monomer is between about 5% and about 50% and the molar ratio of derivatised acryl- or methacryl- carboxylic acid co-monomer is between about 0.1 % and about 15%, wherein the sum of said molar ratios is 100%.
9. The synthetic co-polymer according to any one of aspects1 to 8, wherein said one or more N-alkyl or N,N-dialkyl substituted acrylamide co-monomer is selected from the group of: N-methylacrylamide, N-ethylacrylamide, N-isopropylacrylamide (NiPAAm), N-octylacrylamide, N-cyclohexylacrylamide, N-methyl-N-ethylacrylamide, N-methylmethacrylamide, N-ethylmethacrylamide, N-isopropylmethacrylamide, N,N-dimethylacrylamide, N,N-diethylacrylamide, N,N-dimethylmethacrylamide, N,N-diethylmethacrylamide, N,N-dicyclohexylacrylamide, N-methyl-N-cyclohexylacrylamide, N-acryloylpyrrolidine, N-vinyl-2-pyrrollidinone, N-methacryloylpyrrolidine, and combinations thereof.
10. The synthetic co-polymer according to any one of aspects1 to 9, wherein said one or more hydrophilic co-monomer is a selected from the group of: acrylic acid, methacrylic acid, 2-hydroxyethyl methacrylate (HEMA), N,N-dimethylacrylamide, N,N-diethylacrylamide, 2-[N,N-dimethylammo]ethylacrylamide, 2-[N,N-diethylamino]ethylacrylamide, N,N-diethylmethacrylamide, 2-[N,N-dimethylamino] ethybnethaerylamide, 2-[N,N-diethylamino]ethylmethacrylamide, 2-vinyl-N-pyrrollidone, 2-[N,N-diethylamino] ethylacrylate, 2-[N,N-dimethylamino]ethylacrylate, 2-[N,N-diethylamino]ethylmethacrylate, 2-[N,N-dimethylamino]ethylmethacrylate, and combinations thereof.
11. The synthetic co-polymer according to any one of aspects1 to 10, wherein said one or more acryl- or methacryi-carboxylic acid co-monomer is selected from the group of: acrylic acid, methacrylic acid, and substituted versions thereof, and said cross-linkable moiety is a succinimidyl group, an imidazole, a benzotriazole, a *p*-nitrophenol or 2-(N-morpholino)ethanesulphonic acid.
12. The synthetic co-polymer according to aspect2 that comprises N,N-dimethylacrylamide and N-acryloxysuccinimide.
13. The synthetic co-polymer according to aspect 3 that comprises N-isopropylacrylamide, acrylic acid and N-acryloxysuccinimide.
14. A bio-synthetic matrix comprising:
   (a) the synthetic co-polymer according to any one of aspects1 to 13;
   (b) a bio-polymer, and
   (c) an aqueous solvent,
   wherein said synthetic co-polymer and said bio-polymer are cross-linked through said pendant cross-linkable moiety to form a hydrogel.
15. The bio-synthetic matrix according to aspect 14, wherein the amount of synthetic co-polymer is between about 0.1% and about 30% by weight, the amount of bio-polymer is between about 0.3% and about 50% by weight and the amount of aqueous solvent is between about 20% and about 99.6% by weight.
16. The bio-synthetic matrix according to aspect14 or 15, wherein said bio-polymer is selected from the group of: collagens, denatured collagens, recombinant collagens, gelatin, fibrin-fibrinogen, elastin, glycoprotein, alginate, chitosan, hyaluronic acid, chondroitin sulphate, glycosaminoglycan (proteoglycan), and derivatives thereof.
17. The bio-synthetic matrix according to any one of aspects 14 to 16, further comprising one or more bioactive agent.
18. The bio-synthetic matrix according to aspect17, wherein said one or more bioactive agent is covalently bonded to said synthetic co-polymer through said pendant cross-linkable moiety.
19. The bio-synthetic matrix according to aspect18, wherein said bioactive agent comprises the pentapeptide having the sequence YIGSR.
20. The bio-synthetic matrix according to aspect 17, wherein said one or more bioactive agent is dispersed in said matrix.
21. The bio-synthetic matrix according to any one of aspects 14 to 20, further comprising a plurality of cells dispersed in said matrix.
22. Use of the bio-synthetic matrix according to any one of aspects 14 to 21 as a scaffold for tissue regeneration in an animal in need thereof.
23. Use of the bio-synthetic matrix according to any one of aspects14 to 21 for replacement of damaged or removed tissue in an animal in need thereof.
24. The use according to aspect23, wherein said tissue is skin or part of an organ.
25. The use according to aspect23, wherein said tissue is a cornea or a part of a cornea.
26. Use of the bio-synthetic matrix according to any one of aspects14 to 21 for coating surgical implants.
27. A composition comprising:
   (a) one or more bioactive agent;
   (b) the synthetic co-polymer according to any one of aspects1 to 13;
   (c) a bio-polymer; and
   (d) an aqueous solvent.
28. A composition comprising:
   (a) a plurality of cells;
   (b) the synthetic co-polymer according to any one of aspects1 to 13;
   (c) a bio-polymer, and
   (d) an aqueous solvent.
29. The composition according to aspect27 or 28, wherein the amount of synthetic polymer is between about 0.1% and about 30% by weight, the amount of bio-polymer is between about 0.3% and about 50% by weight and the amount of aqueous solvent is between about 20% and about 99.6% by weight.
30. The composition according to any one of aspects27 to 29, wherein said bio-polymer is selected from the group of: collagens, denatured collagens, recombinant collagens, gelatin, fibrin-fibrinogen, elastin, glycoprotein, alginate, chitosan, hyaluronic acid, chondroitin sulphate, glycosaminoglycan (proteoglycan), and derivatives thereof.
31. The composition according to any one of aspects27 to 30, wherein said synthetic co-polymer and said bio-polymer are cross-linked.
32. The composition according to any one of aspects27 to 31, wherein said bioactive agent is covalently attached to said synthetic co-polymer through said pendant cross-linkable moiety.
33. The composition according to any one of aspects 27 to 30 or 32, which is formulated as an injectable solution, wherein said synthetic co-polymer and said bio-polymer are capable of cross-linking to form a hydrogel *in vivo.*
34. The composition according to any one of aspects27 to 32, which is a pre-formed hydrogel.
35. An implant for use in tissue engineering comprising a pre-formed bio-synthetic matrix, said matrix comprising an aqueous solvent and a bio-polymer cross-linked with the synthetic co-polymer according to any one of aspects to 13.
36. The implant according to aspect35, wherein said bio-polymer is selected from the group of: collagens, denatured collagens, recombinant collagens, gelatin, fibrin-fibrinogen, elastin, glycoprotein, alginate, chitosan, hyaluronic acid, chondroitin sulphate, glycosaminoglycan (proteoglycan), and derivatives thereof.
37. The implant according to aspect 35 or 36, wherein the amount of synthetic polymer is between about 0.1 % and 30% by weight, the amount of bio-polymer is between about 0.3% and 50% by weight and the amount of aqueous solvent is between about 20% and 99.6% by weight.
38. The implant according to any one of aspects35 to 37, wherein said bio-synthetic matrix supports in-growth of nerves.
39. The implant according to any one of aspects35 to 38, further comprising one or more bioactive agent.
40. The implant according to aspect39, wherein said bioactive agent is covalently attached to said synthetic co-polymer through said pendant cross-linkable moiety.
41. The implant according to any one of aspects35 to 40, further comprising a plurality of cells dispersed in said matrix.
42. The implant according to aspect41, wherein said cells are stem cells or precursor cells.
43. Use of the implant according to any one of aspects35 to 40 as an artificial cornea.
44. A process for preparing a synthetic co-polymer comprising:
   (a) dispersing one or more N-alkyl or N,N-dialkyl substituted acrylamide co-monomer, one or more hydrophilic co-monomer and one or more acryl- or methacryl- carboxylic acid co-monomer derivatised to contain a pendant cross-linkable moiety in a solvent in the presence of an initiator;
   (b) allowing said one or more N-alkyl or N,N-dialkyl substituted acrylamide co-monomer, one or more hydrophilic co-monomer and one or more acryl- or methacryl- carboxylic acid co-monomer to polymerise to form a synthetic co-polymer, and
   (c) optionally purifying said synthetic co-polymer.
45. A process for preparing a bio-synthetic matrix comprising the steps of:
   (a) preparing a synthetic co-polymer by the process according to aspect 44;
   (b) dispersing said synthetic co-polymer and a bio-polymer in an aqueous medium; and
   (c) allowing said synthetic co-polymer and said bio-polymer to cross-link to provide said bio-synthetic matrix.
46. The process according to aspect44 or 45, wherein the N-alkyl or N,N-dialkyl substituted acrylamide co-monomer and the hydrophilic co-monomer are the same.
47. The process according to aspect44 or 45, wherein the N-alkyl or N,N-dialkyl substituted acrylamide co-monomer and the hydrophilic co-monomer are different.
48. The process according to aspect45, further comprising mixing said synthetic co-polymer with one or more bioactive agent prior to step (b) and allowing said bioactive agent to cross-link to said synthetic co-polymer through said pendant cross-linkable moiety.
49. The process according to aspect45, further comprising mixing said synthetic co-polymer and said bio-polymer with a plurality of cells in step (b).
50. A synthetic co-polymer produced by the process according to aspect44.
51. A bio-synthetic matrix produced by the process according to aspect45.

## Claims

1. An ocular implant comprising a biosynthetic matrix, said matrix comprising an aqueous solvent and a biopolymer cross-linked with a synthetic co-polymer.

2. The implant of claim 1, wherein the matrix supports cell growth on the implant.

3. The implant of claim 1 or 2, wherein the implant is structured to be placed between a corneal epithelium and a corneal stroma.

4. The implant of claim 1 or 2, wherein the implant is a corneal veneer.

5. The implant of claim 1 or 2, wherein the implant is shaped as a contact lens.

6. The implant of any one of the preceding claims, wherein the biopolymer is cross-linked to the synthetic polymer via a pendant cross-linkable moiety on the synthetic polymer.

7. The implant of any one of the preceding claims, wherein the synthetic polymer is derived from an acrylamide derivative monomer and the biopolymer comprises a protein component.

8. The implant of claim 7, wherein the acrylamide derivative monomer includes a poly(N-iso-propylacrylamide) component.

9. The implant of claim 7, wherein the protein component includes a collagen component.

10. The implant of any one of the preceding claims, wherein the implant is a hydrogel.

11. The implant of claim 10 , wherein the biopolymer is cross-linked to the synthetic polymer so that each polymer is substantially non-extractable from the hydrogel under physiological conditions.

12. The implant of any one of the preceding claims, further comprising a bioactive agent located on or in the implant.

13. The implant according to claim 1 which is a corneal implant.

14. The implant of claim 13, wherein the implant is an artificial cornea.

15. The implant of any one of claims 1, 13 or 14, wherein the synthetic polymer is derived from an acrylamide derivative co-monomer, a hydrophilic co-monomer, and a carboxylic acid co-monomer.

16. The implant of any one of claims 1, 13, 14 or 15, wherein the biopolymer comprises a protein or a carbohydrate.

17. The implant of claim 15 or 16, wherein the acrylamide derivative co-monomer includes a poly(N-iso-propylacrylamide) component.

18. The implant of claim 13, further comprising a bioactive agent cross-linked to a component of the matrix which supports nerve in-growth.

19. The implant of claim 18 , wherein the bioactive agent comprises a protein having a nerve cell attachment motif.

20. The implant of claim 18 or 19, wherein the bioactive agent comprises a peptide.

21. The implant of claim 20, wherein the peptide has an amino acid sequence YIGSR.

22. The implant of any one of claims 18 to 21 wherein the implant is a hydrogel.

23. The implant of claim 22, wherein the hydrogel has a refractive index in a range similar to a refractive index of tear film in a human eye.

24. The implant of claim 22 or 23, wherein the hydrogel includes a plurality of pores having diameters in a range from 140 nm to 190 nm.

25. The implant of any one of claims 22 to 24, wherein the hydrogel has a glucose diffusion permeability greater than a glucose diffusion permeability of a natural stroma of a human eye.

26. The implant of any one of claims 22 to 25, wherein the hydrogel is optically clear.

27. The implant of any one of claims 18 to 26, further comprising living cells located in the matrix.

28. The implant of claim 27, wherein the living cells include ocular cells.

29. The implant of any one of claims 18 to 28, further comprising a reinforcement material provided in the matrix.

30. The implant of any one of claims 18 to 29, wherein the amount of the bioactive agent is effective to enhance touch sensitivity of the implant.
